# EUROPÄISCHE PATENTSCHRIFT

(11) **EP 3 020 824 B1**
(45) Veröffentlichungstag und Bekanntmachung des Hinweises auf die Patenterteilung: **08.11.2017**
(21) Anmeldenummer: 15194954.2
(22) Anmeldetag: 17.11.2015
(51) Int. Cl.: C12Q 1/22, G01N 33/543, G01N 33/569

(54) **KOMPETITIVES IMMUNASSAY-TESTSYSTEM ZUM NACHWEIS EINES PYROGENS**
COMPETITIVE IMMUNOASSAY TEST SYSTEM FOR DETECTING A PYROGEN
SYSTÈME D'IMMUNO-ESSAI DESTINÉ À LA VÉRIFICATION DE PYROGÈNE

(30) Priorität: 17.11.2014 DE 102014223430
(43) Veröffentlichungstag der Anmeldung: 18.05.2016
(73) Patentinhaber: Fraunhofer-Gesellschaft zur Förderung der angewandten Forschung e.V., 80686 München (DE)
(72) Erfinder: Burger-Kentischer, Anke, 70569 Stuttgart (DE); Rupp, Steffen, 70569 Stuttgart (DE)
(74) Vertreter: Schrell, Andreas

(56) Entgegenhaltungen:
- EP-A1- 1 715 341
- WO-A1-97/44665
- CN-A- 103 308 686

## Beschreibung

Die Erfindung betrifft ein kompetitives Immunassay-Testsystem sowie ein Verfahren zum einfachen und schnellen Nachweis von in einer Probe enthaltenen Pyrogenen mittels der Pyrogen-Bindedomäne von Mustererkennungsrezeptoren. Das Testsystem umfasst einen Assayträger, aufweisend mindestens eine immobilisierte Pyrogen-Bindedomäne eines Mustererkennungsrezeptors mit einem markierten, verdrängbaren Liganden, wobei die Verdrängung des Liganden durch ein in der Probe enthaltenes Pyrogen durch eine Farbreaktion der Markierung angezeigt wird. Bevorzugt weist der Assayträger weiterhin mindestens ein immobilisiertes Ligandenfängerprotein auf, welches den verdrängten Liganden bindet und dadurch eine Farbreaktion der Markierung auslöst.

Als Pyrogene werden entzündlich wirkende Stoffe bezeichnet, die Fieber auslösen können. Unterschieden werden hierbei bakterielle Pyrogene, Virus-Pyrogene, Pyrogene aus Pilzen und Pyrogene nicht-biologischen Ursprungs, wie zum Beispiel Metallverbindungen in Elastomeren, Gummiabrieb oder mikroskopische Kunststoffteilchen aus medizintechnischen Produkten oder Pharmaka. Bei den bakteriellen Pyrogenen werden Endotoxine aus der Membran gramnegativer Bakterien, wie zum Beispiel Lipopolysaccharide (LPS), und Bestandteile grampositiver Bakterien, wie zum Beispiel die Teichonsäuren, unterschieden.

In den Körper eingedrungene Pyrogene regen zur Phagozytose befähigte Immunzellen zur Synthese von proinflammatorischen Zytokinen, insbesondere Interleukinen (vor allem IL-1 und IL-6) und Tumornekrosefaktor-α (TNF-α) an, die dann als "eigentliche Pyrogene" das Temperaturzentrum des Körpers so beeinflussen, dass es zu erhöhter Wärmeproduktion und verminderter Wärmeabgabe kommt. Es kommt so zu einer Stimulierung des Immunsystems und normalerweise zur Beseitigung des eingedrungenen Mikroorganismus. Jedoch können pathogene Keime und ihre Produkte, das heißt die Pyrogene, unter Umständen aus einem Infektionsherd in die Blutbahn eindringen und so die Entzündungskaskade systemisch aktivieren, was zu einer nicht mehr kontrolliert ablaufenden systemischen Entzündungsreaktion führt. Der mit dieser Entzündungsreaktion einhergehende klinische Symptomkomplex wird als Sepsis oder umgangssprachlich als "Blutvergiftung" bezeichnet. Seit 1992 werden in diesem Zusammenhang vier separate Schweregrade unterschieden: Systemisches inflammatorisches Response-Syndrom (SIRS), Sepsis, schwere Sepsis und septischer Schock. Die Sepsis als solche ist definiert als ein SIRS mit nachgewiesener Infektion.

Im Verlauf der Sepsis kommt es häufig zu einer lebensbedrohlichen Störung der Vitalfunktionen und zum Versagen eines oder mehrerer Organe (Multiorganversagen). Die Intensivmedizin kann durch vorübergehenden Ersatz oder Unterstützung der Organfunktionen (Beatmung, Nierenersatztherapie, Kreislauftherapie, Gerinnungstherapie) kritische Phasen überbrücken. Trotzdem ist die Sepsis als eine sehr schwere Erkrankung zu werten und die Prognose äußerst ernst: 30-50 % der Erkrankten sterben trotz maximaler Therapie. Der frühestmögliche Therapiebeginn ist entscheidend für ein Überleben. In Deutschland erkranken jährlich 150.000 Menschen an einer Sepsis; 56.300 davon sterben.

Besonders ungünstig ist die Prognose bei spätem Therapiebeginn, nicht lokalisierbarem Infektionsherd oder nicht identifizierbarem Erreger. Von großer Bedeutung für die Einleitung einer zielgerichteten Infektionstherapie ist daher eine schnelle und sichere Identifikation der Erreger oder Erregerspektren in der klinischen Diagnostik in Krankenhäusern.

Sepsis auslösende Erreger sind meist Bakterien, vorwiegend gramnegative Bakterien wie *E. coli*, andere Enterobacterien, *Klebsiella-, Proteus-, Enterobacter-Arten, Pseudomonas aeruginosa, Neisseria meningitidis* und *Bacteroides,* aber auch grampositive Bakterien wie *Staphylococcus aureus, Streptococcus pneumoniae* und andere Streptokokken; seltener Pilze, Viren oder Parasiten. Bei Kontakt der Zellen der unspezifischen Immunabwehr mit Lipopolysacchariden (LPS), also Bestandteilen der bakteriellen Zellwand, Peptidoglykanen oder Lipoteichonsäuren (LTA) wird die angeborene Immunität aktiviert, und es werden in der frühen Phase der Infektion von verschiedenen Immunzellen Zytokine sezerniert. Obwohl diese Zytokine eine wichtige Rolle in der Abwehrreaktion spielen, es werden beispielsweise aktivierte Neutrophile zum Entzündungsort gelockt, führt das Eintreten dieser Zytokine und bakterieller Substanzen in den Blutkreislauf zu einer Kette von ungünstigen pathophysiologischen Ereignissen, welche über Sepsis und septischen Schock zum Tod führen können.

Das Spektrum der auftretenden pyrogenen Substanzen hängt vom Erreger oder Erregerspektrum ab. Bestimmte Erreger bilden erregerspezifische molekulare Strukturen oder Pyrogenmuster, so genannte "pathogen-associated microbial patterns" oder PAMPs, welche von speziellen, so genannten Mustererkennungsrezeptoren (pattern recognition receptors, PRR) wie Toll-like Rezeptoren (TLR), NOD-like Rezeptoren (NLR), RIG I-like (RLR), C-Typ Lectin Rezeptoren (CLR), cytosolischen dsDNA Sensoren (CDSS), Scavenger Rezeptoren, Mannose-binding lectin 2 (MBL-2) und Glucan-Rezeptoren erkannt werden. Unter den PRRs stellen die Toll-like Rezeptoren (TLR) die größte und bekannteste Familie dar.

TLRs sind hoch konservierte Transmembranproteine mit leucinreichen extrazellulären Domänen und einer zytoplasmatischen Domäne aus ungefähr 200 Aminosäuren. Sie gehören aufgrund ihrer Homologie in der Zytoplasmadomäne zur Interleukin-1-Rezeptor/Toll-like-Rezeptor-Superfamilie. Die Extrazellulärdomäne ist direkt an der Erkennung der unterschiedlichen pathogenen Molekülstrukturen beteiligt und stellt somit die Bindedomäne des Rezeptors dar. TLRs werden über die Bindung von PAMPs, also Fremdstrukturen, aktiviert. Bis heute sind zehn verschiedene humane TLRs identifiziert worden. Sie werden auf verschiedenen Zelltypen des Immunsystems exprimiert, überwiegend auf Monozyten, Makrophagen, dendritischen Zellen sowie B- und T-Zellen.

TLR 2 ist essentiell für die Erkennung einer Vielzahl an PAMPs aus grampositiven Bakterien, einschließlich bakterieller Lipoproteine und Lipoteichonsäuren. TLR 3 ist in die Erkennung doppelsträngiger Virus-RNA involviert. TLR 4 wird überwiegend durch LPS gramnegativer Bakterien aktiviert. TLR 5 detektiert bakterielles Flagellin. TLR 7 und TLR 8 erkennen synthetische kleine antivirale Moleküle und einzelsträngige RNA. TLR 9 wurde im Endoplasmatischen Retikulum (ER) nachgewiesen und wird nach Stimulation mit CpG-Motive enthaltender DNA, zum Beispiel CpG-Oligodeoxynucleotide, in die endosomalen/lysosomalen Kompartimente rekrutiert. CpG-Motive sind Bereiche innerhalb eines Nukleinsäure-Stranges, in denen die Bausteine Cytosin (C) und Guanin (G) unerwartet häufig vorkommen (,p' steht für eine Phosphatgruppe, die beide Bausteine ,C' und ,G' verbindet); solche CpG-Motive finden sich besonders häufig im Genom von Bakterien und Viren, nicht aber von Wirbeltieren. Die Spezifität von TLRs wird durch das Zusammenspiel zweier TLRs erweitert, sodass z.B. TLR 2 und TLR 1 zusammen als heterodimeres Molekül in der Lage sind, triacyliertes Lipoprotein zu erkennen. Dimere aus TLR 2 und TLR 6 können diacyliertes Lipoprotein erkennen.

Bei Vorliegen einer septischen Erkrankung oder auch schon bei Verdacht auf eine solche Erkrankung muss eine Therapie frühzeitig erfolgen. Zur Diagnose werden bislang bei einer Sepsis-Indikation Blutkulturen des Patienten für mikrobiologische Untersuchungen angelegt. Dieses Verfahren kostet wertvolle Zeit und führt oft nicht zur Identifikation der Erreger, da es nur bei vitalen Erregern möglich ist. Pyrogene Stoffe der Erreger wie Zellwandbestandteile können mit dieser Methode nicht erfasst werden.

In entsprechenden EU- und FDA-Regularien sind derzeit vier kommerzielle Nachweissysteme für Pyrogene zugelassen: der Kaninchen-Pyrogen-Test (KPT), der Limulus-Amöbozyten-Lysat-Test (LAL), der Immun-Pyrogen-Test (IPT) und der Monozyten-Aktivierungs-Test (MAT).

Der Kaninchen-Pyrogen-Test beruht auf der "Fieberreaktion" der Tiere auf Pyrogene. Es handelt sich um einen Tierversuch, bei welchem den Kaninchen die Prüfsubstanz in die Ohrvene appliziert wird. Um eine Abwehrreaktion des tierischen Körpers gegen die Substanz nachzuweisen wird nach mehreren Stunden rektal Fieber gemessen. Dieser Test ist zeit- und kostenintensiv und mit dem kalkulierten Leiden von Tieren verbunden. Ein weiterer gravierender Nachteil dieses Tests liegt in der hohen Varianz der Ergebnisse, bedingt durch lebende und somit individuelle Organismen. Pyrogene können damit detektiert aber nicht identifiziert werden. Ein Test auf Viren ist ebenfalls nicht möglich. Außerdem ist die Übertragbarkeit auf den Menschen begrenzt, da nicht alle humanpyrogenen Substanzen auch im Kaninchen Fieber auslösen.

Ein weiterer bekannter Test ist der "Limulus-Amöbocyten-Lysat-Test" (LAL, z.B. PyroGene, Lonza; ToxinSensor, GenScript Inc.). Dieser Test nutzt die Tatsache, dass die Hämolymphe des *Limulus polyphenaus* (Pfeilschwanzkrebs) in Gegenwart der LPS gramnegativer Bakterien koaguliert. Dieses Verfahren ist sensitiver und besser standardisierbar als der bekannte Kaninchentest, aber es erfasst nur LPS gramnegativer Organismen (Nachweisgrenze: 3 pg/ml), andere Pyrogene bleiben unerkannt.

Ein weiterer bekannter Test ist schließlich der Immun-Pyrogen-Test, z.B. "Endosafe - IPT" (Fa. Charles River). Er beruht auf der "Fieberreaktion" humaner Zellen auf vorhandene Pyrogene. Es handelt sich um einen humanen Vollbluttest, bei welchem als Antwort auf einen pyrogenen Stoff aus vitalen Blutzellen das Zytokin IL-1 ausgeschüttet wird, das mittels ELISA quantitativ bestimmt werden kann (Nachweisgrenze: 20-50 pg/ml). Dieses System erfasst auch Pyrogene grampositiver Erreger. Der Test ist jedoch mit noch größerem Zeit- und Arbeitsaufwand verbunden. Menschliches Vollblut muss bereitgestellt werden, welches potentiell humanpathogen ist.

Auch der Monozyten-Aktivierungs-Test (MAT, z.B. PyroDetect System, EMD Millipore Corporation; PyroDetect Kit Biotest AG) beruht auf dem Nachweis des von Monozyten gebildeten Zytokin IL-1 mittels ELISA, wobei im Vergleich zum IPT Kryoblut verwendet wird. Eine Spezifikation der Pyrogene ist nicht möglich.

Neben den bisher beschriebenen, in der Pharmacopoeia zugelassenen Tests existieren für die Anwendung in der Forschung weitere in vitro Methoden zur Pyrogendetektion. Hierbei handelt es sich um mammalische Zellen, die TLRs stabil exprimieren und spezifisch die An- oder Abwesenheit von Pyrogenen mithilfe eines Reportergens anzeigen können (z.B. HEK-blue und RAW-blue TLR cells, InvivoGen). Weiterhin offenbart die DE 10 2006 031 483 einen zellulären Pyrogentest, wobei eine transgene NIH-3T3 Zelle mindestens einen TLR und ein Reportergen, das unter der Expressionskontrolle eines durch NF-_{K}B induzierbaren Promotors steht, exprimiert. CN 103 308 686 offenbart ein ELISA-basiertes Detektionsverfahren für LPS. Die bekannten Tests sind zeitaufwändig und setzen ein apparativ gut ausgerüstetes Labor (ELISA-Test, Humanblutverarbeitung, Tierexperiment) sowie Know-how im Umgang mit Zellkultur voraus. Aus diesem Grund sind diese Testsysteme nur für gut ausgebildete Anwender zum Beispiel in der Forschung oder spezialisierten Laboratorien geeignet. Es besteht deshalb der Bedarf an einem schnell und einfach durchzuführenden Testsystem zum Nachweis von Pyrogenen. Es besteht außerdem der Bedarf an einem Testsystem zur Spezifizierung von Pyrogenmustern (PAMPs), um daraus Rückschlüsse auf den Erreger oder das Erregerspektrum ziehen zu können.

Weiterhin müssen medizintechnische Produkte und Pharmaka auf Pyrogenfreiheit untersucht werden, um zu verhindern, dass Pyrogene auf diesem Weg in den Blutkreislauf gelangen. Die Pyrogenfreiheit ist daher eine zwingende Voraussetzung für den Einsatz solcher Produkte am Körper. Es besteht daher Bedarf an verbesserten Tests auf pyrogene Rückstände an medizinischen Geräten, Spendergewebe, injizierbaren Arzneimitteln und Medizinprodukten wie Implantaten oder Instrumente (Katheter, etc.). Auch in der Lebensmittelindustrie und Pharmaindustrie besteht der Bedarf an verbesserten Nachweisen von pyrogenen Substanzen und Keimen und deren Identifikation in Nahrungsmitteln, Nahrungsmittelbestandteilen, Roh- und Ausgangsstoffen für Nahrungs- oder Arzneimittel.

Das der vorliegenden Erfindung zugrunde liegende technische Problem liegt daher vor allem darin, die vorgenannten Nachteile zu überwinden und insbesondere Mittel und Verfahren zum einfachen und schnellen, gleichzeitig jedoch spezifischen Nachweis und Identifikation von Pyrogenen in einer Probe bereitzustellen.

Das technische Problem wird gelöst durch die Bereitstellung eines kompetitiven Immunassay-Testsystems zum Nachweis mindestens eines in einer flüssigen Probe enthaltenen Pyrogens, umfassend einen Assayträger, aufweisend mindestens eine immobilisierte Pyrogen-Bindedomäne mindestens eines Mustererkennungsrezeptors (PRRs, pattern recognition receptor) mit mindestens einem markierten, verdrängbaren Liganden, wobei die Verdrängung des markierten Liganden von der Pyrogen-Bindedomäne des PRRs durch das in der Probe enthaltene Pyrogen durch eine Farbreaktion der Markierung angezeigt wird. Das heißt, bevorzugt sind der markierte, verdrängbare Ligand und die mindestens eine immobilisierte Pyrogen-Bindedomäne so ausgestaltet, dass die Farbreaktion bei Verdrängung des markierten Liganden von der Pyrogen-Bindedomäne stattfindet.

Weiterhin kann der Assayträger in einer bevorzugten Ausführungsform mindestens ein immobilisiertes Ligandenfängerprotein zur Bindung des verdrängten, markierten Liganden aufweisen, wobei die Bindung des Liganden an das Ligandenfängerprotein durch eine Farbreaktion der Markierung angezeigt wird.

Das erfindungsgemäße Immunassay-Testsystem basiert auf einem kompetitiven Immunnachweis, wobei das nachzuweisende Pyrogen eine höhere Bindungsaffinität zur immobilisierten Pyrogen-Bindedomäne des PRRs aufweist als der markierte, verdrängbare Ligand, welcher zunächst an die Bindedomäne gebunden vorliegt und vom nachzuweisenden Pyrogen kompetitiv verdrängt wird. Das heißt, der markierte, verdrängbare Ligand und das nachzuweisende Pyrogen sind verschiedene Substanzen. Der markierte Ligand und das Pyrogen sind allenfalls ähnliche Substanzen, nicht jedoch die gleiche Substanz, die sich lediglich durch das Vorhandensein einer Markierung unterscheidet. Insbesondere unterscheiden sich der verdrängbare Ligand und das nachzuweisende Pyrogen durch ihre Bindungsaffinität zur immobilisierten Pyrogenbindedomäne, wobei das nachzuweisende Pyrogen immer eine höhere Bindungsaffinität aufweist als der verdrängbare Ligand, sodass sichergestellt ist, dass das Pyrogen den Liganden von der Pyrogenbindedomäne, also dem Rezeptor, verdrängen kann, vorzugsweise vollständig und quantitativ verdrängen kann.

Das erfindungsgemäße Testsystem ermöglicht somit vorteilhafterweise, dass auf den Einsatz von Tierversuchen oder zeitlich und apparativ aufwendigen, sehr teuren Bluttests oder Zellkulturverfahren verzichtet werden kann. Weiterhin wird durch die immobilisierten Pyrogen-Bindedomänen individueller PRRs als Bindemoleküle für das entsprechende Pyrogen ein Nachweis spezifischer Erreger und PAMPs ermöglicht, welcher einfach und direkt vor Ort durchgeführt werden kann.

In einer bevorzugten Ausführungsform ist der Assayträger eine Mikrotiterplattenvertiefung, ein Reagenzgefäß, ein Teströhrchen oder ein kapillarflussfähiges Substrat. Die Pyrogen-Bindedomänen des oder der PRRs und bevorzugt die Ligandenfängerproteine werden auf dafür geeigneten Oberflächen des jeweiligen Assayträgers immobilisiert, wie zum Beispiel Boden und/oder Wand einer Mikrotiterplattenvertiefung, eines Reagenzgefäßes oder eines Teströhrchens sowie die Oberfläche eines kapillarflussfähigen Substrats.

Ist der Assayträger als eine Mikrotiterplattenvertiefung, ein Reagenzgefäß oder ein Teströhrchen ausgestaltet, findet der Pyrogennachweis bevorzugt in flüssigem Medium, das heißt bevorzugt einer wässrigen Lösung, insbesondere einem geeigneten Puffer, statt. Der Nachweis kann dann entweder direkt, das heißt durch die bei der Verdrängung des markierten Liganden direkt ausgelöste Farbreaktion, oder indirekt, das heißt durch die Bindung des verdrängten Liganden durch entsprechende Ligandenfängerproteine, erfolgen. Die verdrängten Liganden gelangen hierbei durch Diffusion zu den ebenfalls an Wand und/oder Boden des Assayträgers immobilisierten Ligandenfängerproteinen. Es ist daher erfindungsgemäß bevorzugt nicht vorgesehen, die für den Nachweis notwendigen markierten, verdrängbaren Liganden in Form einer weiteren Komponente oder separaten Lösung zuzugeben. Weiterhin ist ein inverser Nachweis, das heißt ein Nachweis von in der Probe enthaltenem Pyrogen durch eine Reduktion der Färbung oder Farbreaktion, erfindungsgemäß bevorzugt nicht vorgesehen. Für die Durchführung des erfindungsgemäßen Verfahrens werden somit bevorzugt auch keine Farbskalen benötigt, die die Intensität der Färbung mit dem Pyrogengehalt in der Probe korrelieren, vielmehr zeigt eine Farbreaktion direkt das Vorhandensein eines oder mehrerer Pyrogene in der Probe an.

In einer besonders bevorzugten Ausführungsform der vorliegenden Erfindung ist der Assayträger ein kapillarflussfähiges Substrat, welches in Kapillarflussrichtung folgende, jeweils voneinander beabstandete Bereiche aufweist:
a) einen Probeaufgabebereich,
b) einen Reaktionsbereich, aufweisend die mindestens eine immobilisierte Pyrogen-Bindedomäne mindestens eines PRRs mit dem mindestens einen markierten, verdrängbaren Liganden und
c) einen Analysebereich, aufweisend das mindestens eine immobilisierte Ligandenfängerprotein zur Bindung des verdrängten, markierten Liganden.

In dieser Ausführungsform beruht das erfindungsgemäße Testsystem auf einem kompetitiven immunchromatographischen Nachweis des Pyrogens. Da es sich dabei um eine spezielle Ausführungsform eines kompetitiven Immunnachweises handelt, gelten hierfür bevorzugt ebenfalls die vorstehend für einen kompetitiven Immunnachweis ausgeführten Bedingungen, sofern auf ein kapillarflussfähiges Substrat anwendbar.

Das erfindungsgemäße Testsystem detektiert Pyrogene in flüssigen Proben. Bevorzugt handelt es sich bei der flüssigen Probe um eine klinische Probe, eine medizintechnische Probe, eine pharmazeutische Probe, eine lebensmitteltechnologische Probe oder eine Umweltprobe. Besonders bevorzugt handelt es sich bei einer klinischen Probe um eine Probe menschlichen oder tierischen Ursprungs. Besonders bevorzugt handelt es sich bei einer Umweltprobe um eine Wasserprobe, insbesondere eine Trinkwasserprobe oder eine Probe aus einem Badegewässer. Selbstverständlich können mit dem erfindungsgemäßen Immunassay-Testsystem alle Arten von Proben analysiert werden, in denen die Anwesenheit von Pyrogenen vermutet wird, solange sie in flüssiger Form und in ausreichendem Volumen vorliegen.

Bevorzugt umfasst das erfindungsgemäß bevorzugte kapillarflussfähige Substrat zusätzlich zum Probeaufgabebereich a), dem Reaktionsbereich b) und dem Analysebereich c) einen Kontrollverbindungsbereich b'), aufweisend mindestens eine adsorbierte, markierte Kontrollverbindung, die nicht an die Pyrogen-Bindedomäne des PRRs oder das Ligandenfängerprotein bindet und d) einen Kontrollbereich, aufweisend mindestens ein immobilisiertes Kontrollverbindungsfängerprotein zur Bindung der mindestens einen, markierten Kontrollverbindung.

Über den Probeaufgabebereich a) gelangt eine gegebenenfalls pyrogenhaltige Probe oder Lösung durch Kapillarfluss zum Reaktionsbereich b). Im Reaktionsbereich b) befindet sich mindestens eine an das Substrat gebundene, immobilisierte Pyrogen-Bindedomäne mindestens eines PRRs, an welche markierte, verdrängbare Liganden gebunden sind. Das nachzuweisende Pyrogen weist eine höhere Bindungsaffinität zur immobilisierten Pyrogen-Bindedomäne des PRRs auf als der markierte, verdrängbare Ligand. Dies ist von Bedeutung, da der Nachweis des Pyrogens auf einer kompetitiven Verdrängung des markierten Liganden beruht, der dann im Analysebereich spezifisch, bevorzugt durch eine Farbreaktion, nachgewiesen wird. Da das spezifische Pyrogen eine höhere Bindungsaffinität zur immobilisierten Pyrogen-Bindedomäne des PRRs aufweist, werden die markierten Liganden von der Pyrogen-Bindedomäne des PRRs verdrängt und durch Kapillarfluss weitergetragen. Das Pyrogen wird daher im Analysebereich indirekt durch die Bindung des verdrängten markierten Liganden nachgewiesen. Die bevorzugt vorhandenen mobilen, das heißt nicht immobilisierten Kontrollverbindungen werden ebenfalls mit dem Kapillarfluss weitergetragen, da sie lediglich an das Substrat adsorbiert sind und so anzeigen können, ob die flüssige Probe bis zum Analysebereich vorgedrungen ist. Die erfolgreiche Durchführung des Tests kann so kontrolliert werden, um falsch negative Ergebnisse zu vermeiden. Im Analysebereich werden die von der Pyrogen-Bindedomäne des PRRs durch das spezifische Pyrogen verdrängten markierten Liganden durch spezifische, im Analysebereich immobilisierte Ligandenfängerproteine gebunden und so nachgewiesen. Dies kann bevorzugt durch eine Farbreaktion angezeigt werden, die erst durch die Bindung der markierten Liganden an die Ligandenfängerproteine im Analysebereich ausgelöst wird. Alternativ ist es bevorzugt auch möglich, dass die Farbmarkierung der verdrängbaren Liganden bereits im Reaktionsbereich sichtbar ist, im Falle einer Verdrängung durch in der Probe enthaltenes Pyrogen im Kapillarfluss weitertransportiert wird und dann durch die Bindung an die Ligandenfängerprotein im Analysebereich aufkonzentriert wird. Bevorzugt wird die Kontrollverbindung durch Kontrollverbindungsfängerproteine gebunden und nachgewiesen.

Im Zusammenhang mit der vorliegenden Erfindung werden unter dem Begriff "Pyrogen" human- und/oder tierpathogene Bakterien, Viren, Pilze und Parasiten sowie deren Bestandteile, molekulare Strukturen und PAMPs verstanden, aber auch Substanzen nicht-biologischen Ursprungs, zum Beispiel Metallverbindungen in Elastomeren, Gummiabrieb oder mikroskopische Kunststoffteilchen aus medizintechnischen Produkten oder Pharmaka.

Erfindungsgemäß bevorzugt ist das nachzuweisende Pyrogen mindestens eines ausgewählt aus der Gruppe bestehend aus bakteriellen Lipoproteinen, bakteriellen Lipopeptiden, bakteriellen Peptidoglykanen, bakteriellen Lipoteichonsäuren, Zymosan aus Hefen, doppelsträngiger Virus-RNA, bakteriellen Lipopolysacchariden (LPS), bakteriellem Flagellin, nicht methylierter CpG-enthaltender bakterieller oder viraler DNA, einzelsträngiger Virus-RNA und menschlichem Heatshockprotein 60.

Zum Nachweis eines oder mehrerer der vorstehend spezifizierten Pyrogene werden im erfindungsgemäßen Immunassay-Testsystem die Pyrogen-Bindedomänen mindestens eines PRRs eingesetzt. Die Pyrogen-Bindedomäne eines PRRs umfasst bevorzugt diejenigen Bereiche des jeweiligen Rezeptors, die für die Erkennung eines Pyrogens, das heißt seiner Bindung, verantwortlich sind. Die Pyrogen-Bindedomäne stellt daher nur einen Teil des gesamten Rezeptors dar, der jedoch an der direkten Erkennung der Pyrogene beteiligt ist. Die entsprechenden Rezeptordomänen können entweder durch entsprechende Enzyme vom gesamten Protein abgeschnitten, aus überexprimierenden Zellen isoliert, über entsprechend transfizierte Zellen als verkürztes Protein exprimiert oder mittels zellfreier Proteinsynthese mit Hilfe des Transkriptions- und Translationsapparat von lysierten Zellen in vitro exprimiert und anschließend aufgereinigt werden.

Selbstverständlich ist es auch möglich, den gesamten PRR auf dem Assayträger, bevorzugt im Reaktionsbereich b) des kapillarflussfähigen Substrats zu immobilisieren. Es kann daher erfindungsgemäß vorgesehen sein, dass der Reaktionsbereich b) mindestens einen immobilisierten PRR mit markierten, verdrängbaren Liganden aufweist.

Die Pyrogen-Bindedomäne eines PRRs oder das gesamte Rezeptorprotein können über entsprechend transfizierte Zellen exprimiert und aufgereinigt werden. Die so oder auf anderem Wege erhaltenen Proteine können entweder ungerichtet, beispielsweise über Physisorption unter Ausnutzung von elektrostatischen Wechselwirkungen, Van-der-Waals-Wechselwirkungen, ionischen Wechselwirkungen, Wasserstoff-Brückenbindungen und hydrophoben Wechselwirkungen, oder gerichtet auf dem Assayträger immobilisiert werden, beispielsweise mittels Affinitätsadsorption mit Hilfe sogenannter "tags". Dabei wird das entsprechende "tag" bei der Expression am der Pyrogen-Bindungsdomäne entgegensetzten Proteinterminus angeknüpft, so dass bei der Affinitätsadsorption stets der Proteinterminus mit der Pyrogen-Bindedomäne von der Trägeroberfläche weg weist. Die "tags" weisen dabei eine hohe Bindungsaffinität zu ihrem jeweiligen Bindungspartner auf. Beispiele solcher Affinitätspaarungen sind Streptavidin/Avidin und Biotin, Maltose und Maltose-bindendes Protein (MBP), Glutathion und Glutathion-S-Transferase (GST) sowie Streptavidin und Histidin. Die Proteine können ferner mittels Chemisorption über kovalente Bindungen an funktionellen Seitengruppen der Aminosäuren wie Amino-, Carboxyl, Hydroxyl- und Thiolgruppen über Isoharnstoffverbindungen, Diazoverbindungen, Peptidbindungen oder Alkylierungsreaktionen auf dem Assayträger immobilisiert werden.

Erfindungsgemäß kann auch vorgesehen sein, dass intakte Zellen, die den oder die entsprechenden PRRs an der Zelloberfläche exprimieren, oder Lysate von Zellen, die PRRs exprimieren, direkt mithilfe des Inkjet-Druckverfahrens als komplette Zelle oder Zelllysat auf den Assayträger, bevorzugt das kapillarflussfähige Substrat gedruckt und so immobilisiert werden. Hierzu werden Protein-basierte, insbesondere Gelatinederivate aufweisende Biotinten verwendet.

In einer bevorzugten Ausführungsform der vorliegenden Erfindung ist die mindestens eine, immobilisierte Pyrogen-Bindedomäne des PRRs ausgewählt aus der Gruppe bestehend aus den Pyrogen-Bindedomänen eines Toll-like-Rezeptors (TLR), eines NOD-like Rezeptors (NLR), eines RIG I-like (RLR) Rezeptors, eines C-Typ Lectin Rezeptors (CLR), eines cytosolischen dsDNA Sensors (CDSS), eines Scavenger Rezeptors, eines Mannose-binding lectin 2 Rezeptors (MBL-2), eines Glucan-Rezeptors und Kombinationen davon. Besonders bevorzugt ist die mindestens eine, immobilisierte Pyrogen-Bindedomäne die Pyrogen-Bindedomäne eines TLRs. In bevorzugter Ausführungsform ist die mindestens eine, immobilisierte Pyrogen-Bindedomäne eines oder mehrerer TLRs ausgewählt aus der Gruppe bestehend aus TLR 1, TLR 2, TLR 3, TLR 4, TLR 5, TLR 6, TLR 7, TLR 8, TLR 9 und Heterodimeren davon. Vorteilhafterweise können daher je nach Auswahl oder Zusammenstellung des oder der immobilisierten Pyrogen-Bindedomäne(n) verschiedener PRRs, insbesondere TLRs oder Heterodimeren derselben verschiedene Pyrogene spezifisch, das heißt einzeln, oder auch verschiedene Pyrogengruppen detektiert werden.

In einer bevorzugten Ausführungsform der Erfindung sind die Pyrogen-Bindedomänen von mindestens zwei verschiedenen PRRs, insbesondere TLRs, auf dem Assayträger, insbesondere im Reaktionsbereich b) des kapillarflussfähigen Substrats, immobilisiert. Entsprechend sind an die Pyrogen-Bindedomänen mindestens zwei markierte, verdrängbare Liganden gebunden und entsprechend mindestens zwei Ligandenfängerproteine vorgesehen, die für jeweils einen Liganden spezifisch sind. Die mindestens zwei Liganden können mit der gleichen oder unterschiedlichen Farbmarkierung(en) markiert sein.

Bevorzugt sind in dieser Ausführungsform auf dem kapillarflussfähigen Substrat die Pyrogen-Bindedomänen von mindestens zwei verschiedenen PRRs, jeweils mit markierten Liganden, in einem Reaktionsbereich b) immobilisiert und die mindestens zwei Ligandenfängerproteine in einem Analysebereich c). Es kann jedoch auch vorgesehen sein, dass das erfindungsgemäße Testsystem jeweils einen Reaktionsbereich für die immobilisierte Pyrogen-Bindedomäne eines PRRs und jeweils einen Analysebereich für die entsprechenden Ligandenfängerproteine aufweist. Gleiches gilt dann für den Probeaufgabebereich a) und, falls vorhanden, den Kontrollverbindungsbereich b') und den Kontrollbereich d).

In einer besonders bevorzugten Ausführungsform der vorliegenden Erfindung ist die mindestens eine, immobilisierte Pyrogen-Bindedomäne des PRRs die Pyrogen-Bindedomäne des TLR 4. In dieser Ausführungsform können mit dem erfindungsgemäßen Immunassay-Testsystem in der Probe enthaltene Lipopolysaccharide (LPS) als Pyrogene aus gramnegativen Bakterien nachgewiesen werden.

In einer weiteren besonders bevorzugten Ausführungsform der vorliegenden Erfindung ist die mindestens eine, immobilisierte Pyrogen-Bindedomäne des PRRs die Pyrogen-Bindedomäne des TLR 9. In dieser Ausführungsform kann mit dem erfindungsgemäßen Immunassay-Testsystem in der Probe enthaltene nicht methylierte CpG-enthaltende bakterielle oder virale DNA nachgewiesen werden.

Der markierte, verdrängbare Ligand besitzt eine geringere, bevorzugt mittlere, Bindungsaffinität zur Pyrogen-Bindedomäne des PRRs im Vergleich zum für den PRR spezifischen Pyrogen. Dadurch wird sichergestellt, dass der markierte Ligand vom nachzuweisenden Pyrogen vom PRR, insbesondere der Pyrogen-Bindedomäne des PRRs, verdrängt werden kann. Der Ligand ist daher bevorzugt eine Pyrogenähnliche Substanz oder Verbindung, die zwar an die Pyrogen-Bindedomäne binden kann, jedoch mit geringerer Bindungsaffinität als das eigentliche Pyrogen. Bevorzugt ist der verdrängbare Ligand eine niedermolekulare Verbindung, besonders bevorzugt ein Derivat der Substanzen ausgewählt aus der Gruppe bestehend aus Pyrimidin, Quinolin, Sulfonamid, Carboxamid, Phenanthrolin, Piperarzin, Benzensulfonamid, Pyrazin, Naphtyridin und Morpholin; ein Peptid, bevorzugt bestehend aus 7 bis 17 Aminosäuren; ein einzelsträngiges CpG-DNA-Oligodesoxynukleotid; ein Adenin-Analogon; eine Polyinosinic:Polycytidylic acid; ein Lipoprotein; ein Lipid; ein Muramyldipeptid; ein Hyaluronsäurefragment; ein doppelsträngiges DNA-Nukleotid; ein Kohlenhydrat, besonders bevorzugt ein Derivat von Mannose oder Fucose; ein Peptidoglycan oder ein doppelsträngiges RNA-Nukleotid. Bevorzugt kann die Bindungsaffinität des Liganden weiterhin durch Einstellung geeigneter Bedingungen auf oder in dem Assayträger, wie zum Beispiel pH-Wert und/oder Auswahl und Konzentration geeigneter Puffersalze entsprechend beeinflusst und eingestellt werden.

Der verdrängbare Ligand und, in bevorzugter Ausführungsform die Kontrollverbindung, sind markiert, insbesondere farbmarkiert. Besonders bevorzugt sind der verdrängbare Ligand und, falls vorhanden, die Kontrollverbindung entweder mit der gleichen oder unterschiedlichen Farbmarkierung(en) ausgewählt aus der Gruppe bestehend aus Fluoreszenzfarbstoffen, insbesondere Fluoresceinisothiocyanat (FITC), Fluorescein, Rhodamin, ATTO-Farbstoffen, Texas Red, Phycoerythrin-Derivaten, Cyanin-Fluoreszenzfarbstoffen, Tetramethyl-rhodamine 5-Carboxamido-(6-Azidohexanyl) (TAMRA), Alexa Fluor 488; farbigen Latexpartikeln, Rußpartikeln, magnetischen Partikeln, Quantum dots, kolloidalen Selenpartikeln, Goldnanopartikeln, Goldclustern, kolloidalen Goldpartikeln, kolloidalen Selenpartikeln; Enzymen, insbesondere Meerrettich-Peroxidase (HRP), Alkalische Phosphatase (AP), Glucoseoxidase (GOx), β-D-Galactosidase (β-Gal), Glucose 6-Phosphat Dehydrogenase (G6PD); chemilumineszenten Verbindungen, insbesondere Acridiniumester, Acridiniumsulfonamid, Isoluminol; Streptavidin, Avidin und Biotin markiert.

Der Nachweis der Markierung erfolgt jeweils markierungsspezifisch. Direkte Farbmarkierungen wie Fluoreszenzfarbstoffe oder Quantum Dots werden durch eine Zu- oder Abnahme der Fluoreszenzintensität bei einer geeigneten Exitationswellenlänge und einer geeigneten Emissionswellenlänge gemessen. Farbige Partikel wie Latexpartikel, Rußpartikel oder kolloidale Selenpartikel können ohne zu Hilfenahme von weiteren Auswerteinstrumenten visuell detektiert werden, wenn sie ortsaufgelöst anhand der Bindung des Liganden an die Ligandenfängerproteine aufkonzentriert werden. Magnetische Partikel können mittels Änderungen der Remanenz und der Entspannung in magnetischen Materialien nachgewiesen werden. Goldnanopartikel werden bevorzugt über oberflächenverstärkte Ramanspektroskopie nachgewiesen. Der Nachweis mittels Enzymen wird über den Umsatz geeigneter farbiger Enzymsubstrate und einem damit verbundenen Farbumschlag photometrisch oder mittels Intensitätsänderungen der Chemilumineszenz nachgewiesen. Besonders bevorzugte Substrate der HRP basieren auf chromogenen Substraten wie 3,3',5,5'-Tetramethylbenzidin (TMB), 3,3'-Diaminobenzidin (DAB), 2,2'-Azino-bis(3-ethylbenzothiazolin-6-schwefelsäure) (ABTS), o-Phenylendiamindihydrochlorid (OPD) oder chemilumineszenten Substraten wie enhanced chemiluminescence substrate (ECL). Bevorzugte Substrate der AP basieren auf 5-Bromo-4-chloro-3-indolylphosphat (BCIP), para-Nitrophenylphosphat (pNPP) oder Acridinsubstraten. Bevorzugte Substrate der GOx sind Nitro-blau-tetrazoliumsalze und 5-Bromo-4-chloro-3'-indolyphosphat. Bevorzugte Substrate der β-Gal sind o-Nitrophenyl-β-D-galactopyranosid und 5-Brom-4-chlor-3-indoxyl-β-D-galactopyranosid. Bevorzugte Substrate der G6PD sind 4-Nitrophenyl-α-D-glucopyranosid, 5-Bromo-4-chloro-3-indolyl-β-D-glucuronid, 4-Nitrophenyl-β-D-glucuronid. Chemilumineszente Verbindungen werden über die Änderung der Lumineszenz in geeigneten Auswertegeräten ausgewertet.

Bei der Markierung mit dem System Streptavidin/Avidin und Biotin erfolgt der Nachweis indirekt über eine Konjugation des Streptavidin oder Avidin mit geeigneten Enzymen wie HRP, AP oder Fluoreszenzfarbstoffen wie Fluorescein oder Rhodamin und der vorstehend beschriebenen Auswertung.

Die Farbmarkierung wird über Standardkopplungsmethoden, die dem Fachmann geläufig sind, an den Liganden und gegebenenfalls die Kontrollverbindung gebunden.

In bevorzugter Ausführungsform sind die Ligandenfängerproteine spezifische Antikörper, insbesondere monoklonale oder polyklonale Antikörper, die den verdrängbaren Liganden spezifisch binden. Durch die Bindung des markierten Liganden wird eine Farbreaktion ausgelöst, die die Bindung des verdrängten Liganden anzeigt. In einer weiteren bevorzugten Ausführungsform sind die Ligandenfängerproteine monoklonale oder polyklonale Antikörper, die die Farbmarkierung des verdrängbaren Liganden spezifisch binden.

In einer bevorzugten Ausführungsform können auf dem kapillarflussfähigen Substrat die Ligandenfängerproteine auch Pyrogen-Bindedomänen der PRRs sein. In besonders bevorzugter Ausführungsform können also die im Reaktionsbereich des kapillarflussfähigen Substrats immobilisierten Pyrogen-Bindedomänen des oder der PRRs als Ligandenfängerproteine verwendet und ebenfalls im Analysebereich immobilisiert werden. In einer bevorzugten Ausführungsform können die Ligandenfängerproteine spezifische Antikörper und Pyrogen-Bindedomänen der PRRs sein.

Die Kontrollverbindung ist bevorzugt eine Substanz oder ein Molekül, das eine möglichst ähnliche Struktur im Vergleich zum verdrängbaren Liganden aufweist, jedoch nicht an die Pyrogen-Bindedomäne des PRRs bindet. Durch die möglichst ähnliche Struktur der Kontrollverbindung und des verdrängbaren Liganden wird vorteilhafterweise ein möglichst ähnliches Laufverhalten der beiden Substanzen im Kapillarfluss des Substrats ermöglicht. Dadurch kann eine besonders exakte Kontrolle des Tests erfolgen, da die Kontrollverbindung und der verdrängbare Ligand in etwa gleich schnell im Kapillarfluss transportiert werden und so sichergestellt werden kann, dass eine positive Kontrollreaktion auch aussagekräftig ist und ein gegebenenfalls verdrängter Ligand bis zum Analysebereich transportiert wurde. Die Kontrollverbindung ist bevorzugt eine Substanz, die nicht an die Pyrogen-Bindedomäne des PRRs bindet, beispielsweise eine niedermolekulare Verbindung, besonders bevorzugt ein Derivat der Substanzen ausgewählt aus der Gruppe bestehend aus Pyrimidin, Quinoxalin, Quinolin, Benzen, Quinazolin Imidazolin, Sulfonamid und Carboxamid; ein Peptid, bevorzugt bestehend aus 7 bis 17 Aminosäuren; ein einzelsträngiges CpG-DNA-Oligodesoxynukleotid; ein Adenin-Analogon; eine Polyinosinic:Polycytidylic acid; ein Lipoprotein; ein Lipid; ein Muramyldipeptid; ein Hyaluronsäurefragment; ein doppelsträngiges DNA-Nukleotid; ein Kohlenhydrat, besonders bevorzugt ein Derivat von Mannose oder Fucose; ein Peptidoglycan oder ein doppelsträngiges RNA-Nukleotid.

Bevorzugt sind die Kontrollverbindungsfängerproteine spezifische Antikörper, die die Kontrollverbindung spezifisch binden. Da die Kontrollverbindung markiert, insbesondere farbmarkiert ist, wird durch die Bindung der Kontrollverbindung an die Kontrollverbindungsfängerproteine eine Farbreaktion ausgelöst und die Bindung sichtbar gemacht. In einer weiteren bevorzugten Ausführungsform sind die Kontrollverbindungsfängerproteine monoklonale oder polyklonale Antikörper, die die Farbmarkierung der Kontrollverbindung spezifisch binden.

In einer bevorzugten Ausführungsform sind die Bereiche a), b) und c) auf dem kapillarflussfähigen Substrat jeweils voneinander beabstandet. In bevorzugter Ausführungsform überlappen sich die verschiedenen Bereiche nicht, sondern weisen einen Abstand zwischen einander auf oder grenzen aneinander an.

In einer bevorzugten Ausführungsform weist das kapillarflussfähige Substrat die Bereiche a), b), b'), c) und d) auf. Bevorzugt sind dann die Bereiche a), b), c) und d) jeweils voneinander beabstandet und die Bereiche b) und b') können voneinander beabstandet sein oder bevorzugt miteinander überlappen. In dieser Ausführungsform weisen also die Bereiche a), b), c) und d) einen Abstand zwischen einander auf oder grenzen einander an und die Bereiche b) und b') weisen einen Abstand zwischen einander auf, grenzen aneinander an oder überlappen miteinander. Bevorzugt überlappen die Bereiche b) und b') vollständig miteinander. In bevorzugter Ausführungsform stellen die Bereiche b) und b') einen Bereich dar, sodass die immobilisierte Pyrogen-Bindedomäne mindestens eines Mustererkennungsrezeptors mit dem markierten, verdrängbaren Liganden sowie die adsorbierte markierte Kontrollverbindung sich in ein und demselben Bereich des kapillarflussfähigen Substrats befinden.

Weiterhin bevorzugt sind die verschiedenen Bereiche in der folgenden Reihenfolge auf dem kapillarflussfähigen Substrat angeordnet: a), b) und c) oder a), b), b') und d), wobei die Bereiche b) und b') überlappen, insbesondere vollständig überlappen, können oder getrennt voneinander vorliegen.

In einer bevorzugten Ausführungsform umfasst das erfindungsgemäße Immunassay-Testsystem als Assayträger ein kapillarflussfähiges Substrat, vorzugsweise besteht aus einem kapillarflussfähigen Substrat, das die Bereiche a), b), c) und bevorzugt b') und d) aufweist. Unter Substrat wird im Zusammenhang mit der vorliegenden Erfindung eine feste Matrix, insbesondere Trägermatrix, verstanden, die flüssige Proben aufnehmen kann und einen Kapillarfluss der Probe, insbesondere von in der Probe enthaltenen Substanzen, wie zum Beispiel Pyrogenen, erlaubt und weiterhin zur Immobilisierung von Rezeptorproteinen, Antikörpern oder auch Zellen oder Zelllysaten geeignet ist. Optional kann das Substrat geträgert vorliegen, das heißt durch ein festeres Material, nämlich den Träger, stabilisiert werden. Vorzugsweise kann das Substrat selbst rund, oval, quadratisch oder in Form eines Streifens sein, das heißt in Form eines Kreises, Ovals, Quadrates oder eines Streifens ausgebildet sein. Besonders bevorzugt ist das Substrat in Form eines Streifens, also rechteckig, ausgebildet. Darauf sind die einzelnen Bereiche bevorzugt aufeinanderfolgend, bevorzugt entlang der Längsachse des Rechtecks, angeordnet.

Besonders bevorzugt ist das kapillarflussfähige Substrat ein poröses Substrat aus Nitrocellulose, Celluloseacetat, ladungsmodifiziertem Nylon, Polyethersulfon, Polyvinylidenfluorid, Glasfasermembranen, Sol-Gel-Schichten aus Aluminiumoxid, Titandioxid, Siliziumdioxid oder ähnlichen Materialien oder Kombinationen daraus. Besteht das Substrat aus einer Kombination verschiedener Materialien können diese bevorzugt aneinander stoßen, ineinander übergehen oder einander überlappen. Besonders bevorzugt sind die Substrate ausgewählt aus Tabelle 1, nachstehend.

Bevorzugt umfasst das erfindungsgemäße Immunassay-Testsystem als Assayträger also ein kapillarflussfähiges Substrat oder Kombinationen mehrerer kapillarflussfähiger Substrate, bevorzugt besteht der Assayträger aus dem oder den kapillarflussfähigen Substrat(en). Es kann jedoch erfindungsgemäß auch vorgesehen sein, dass das kapillarflussfähige Substrat in einer Vorrichtung, zum Beispiel einem Gehäuse, enthalten ist, die die Handhabung erleichtert. Bevorzugt weist die Vorrichtung Aussparungen auf, die beispielsweise das Auftragen der Probe auf den Probeaufgabebereich und das Auslesen des Analyse- und, falls vorhanden, des Kontrollbereichs erlauben.

Die vorliegende Erfindung betrifft weiterhin ein Verfahren zum Nachweis mindestens eines in einer flüssigen Probe enthaltenen Pyrogens umfassend die Verfahrensschritte, vorzugsweise bestehend aus den Verfahrensschritten:
i) Bereitstellen eines erfindungsgemäßen Testsystems,
ii) Inkontaktbringen einer flüssigen Probe mit dem Assayträger und
iii) Auswerten der Farbreaktion.

Sofern der Assayträger des erfindungsgemäßen Testsystems eine Mikrotiterplattenvertiefung, ein Reagenzgefäß oder ein Teströhrchen ist, wird unter "Inkontaktbringen" der flüssigen Probe mit dem Assayträger das Einbringen der Probe in den Reaktionsbehälter, das heißt die Mikrotiterplattenvertiefung, das Reagenzgefäß oder das Teströhrchen, verstanden.

Erfindungsgemäß kann durch das Einbringen der flüssigen Probe in die Mikrotiterplattenvertiefung, das Reagenzgefäß oder das Teströhrchen ein in der Probe enthaltenes Pyrogen zu den immobilisierten Pyrogen-Bindedomänen des PRRs diffundieren und den markierten Liganden von der Bindedomäne verdrängen. Entweder wird durch die Verdrängung direkt eine Farbreaktion der Markierung ausgelöst oder der verdrängte Ligand wird indirekt von ebenfalls immobilisierten Ligandenfängerproteinen gebunden, wodurch eine Farbreaktion der Markierung ausgelöst wird.

In einer bevorzugten Ausführungsform des erfindungsgemäßen Verfahrens ist der Assayträger ein kapillarflussfähiges Substrat und die flüssige Probe wird in Schritt ii) auf den Probeaufgabebereich a) aufgebracht oder der Probeaufgabebereich des Substrats wird in die Probe eingetaucht. Dabei werden Bedingungen bereitgestellt und aufrechterhalten, die zu einem Kapillarfluss im Substrat vom Probeaufgabebereich a) bis zum Analysebereich c), bevorzugt bis zum Kontrollbereich d), führen.

Bevorzugt werden die Bedingungen, die zu einem Kapillarfluss im Substrat führen für einen Zeitraum von mindestens einer Minute, insbesondere eine Minute bis 24 Stunden, insbesondere 10 Minuten bis 12 Stunden, insbesondere 15 Minuten bis 1 Stunde, vorzugsweise 20 Minuten bis drei Stunden, vorzugsweise 30 Minuten bis zwei Stunden, aufrechterhalten.

Erfindungsgemäß wird durch das Aufbringen der flüssigen Probe auf ein kapillarflussfähiges Substrat ein in der Probe enthaltenes Pyrogen durch Kapillarfluss in Richtung des Reaktionsbereichs b) transportiert, bindet dort an die immobilisierte Pyrogen-Bindedomäne des PRRs und verdrängt dadurch den markierten Liganden von der immobilisierten Pyrogen-Bindedomäne des PRRs, welcher durch den Kapillarfluss weiter zum Analysebereich c) transportiert, dort durch das immobilisierte Ligandenfängerprotein gebunden und nachgewiesen wird.

Bevorzugt wird eine im Kontrollverbindungsbereich b') adsorbierte Kontrollverbindung durch den Kapillarfluss bis zum Kontrollbereich d) transportiert, dort durch das immobilisierte Kontrollverbindungsfängerprotein gebunden und nachgewiesen.

Alle in der vorliegenden Beschreibung verwendeten Begriffe, wie zum Beispiel Pyrogen-Bindedomäne, Mustererkennungsrezeptor (PRR oder TLR), verdrängbarer Ligand, Ligandenfängerprotein, Kontrollverbindung und Kontrollverbindungsfängerprotein, umfassen auch die Mehrzahl der entsprechenden Komponenten.

Weitere bevorzugte Ausführungsformen der vorliegenden Erfindung ergeben sich aus den Unteransprüchen.

Die vorliegende Erfindung wird anhand eines Beispiels und der folgenden Figuren näher erläutert.

Die Figuren zeigen:

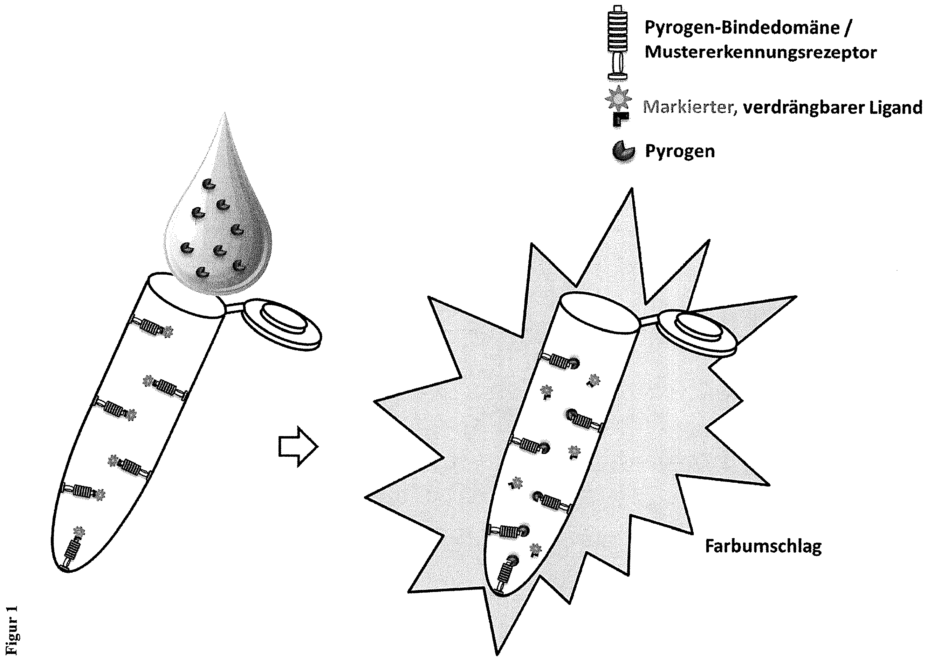

**Figur 1** zeigt das erfindungsgemäße Immunassay-Testsystem, wobei der Assayträger als Teströhrchen ausgestaltet ist und Pyrogen-Bindedomänen eines PRRs (Mustererkennungsrezeptor) auf der inneren Oberfläche des Teströhrchens immobilisiert sind.

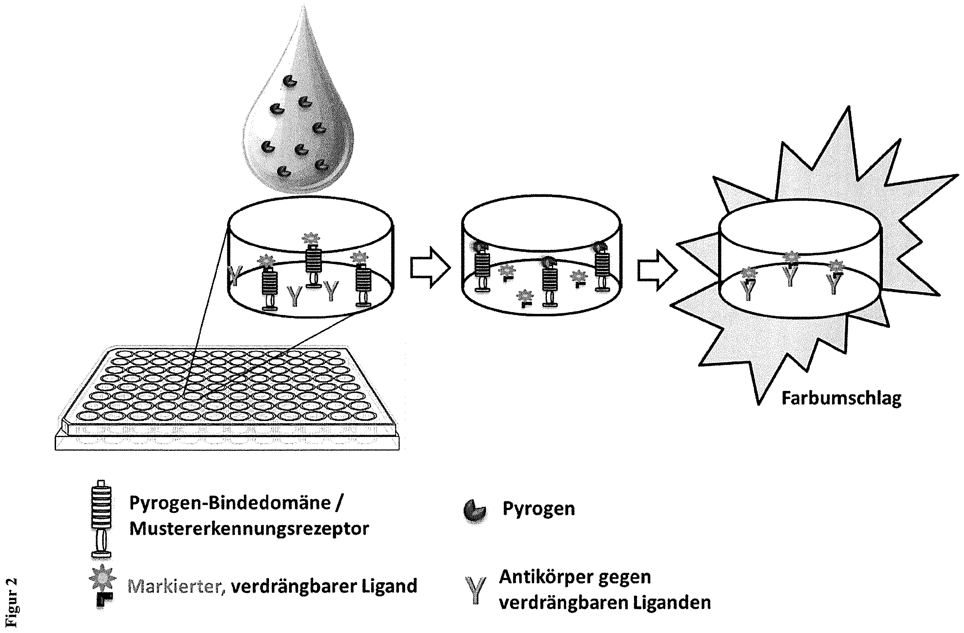

**Figur 2** zeigt das erfindungsgemäße Immunassay-Testsystem, wobei der Assayträger als Mikrotiterplattenvertiefung ausgestaltet ist und Pyrogen-Bindedomänen eines PRRs und Ligandenfängerproteine (Antikörper) auf dem Boden der Vertiefung immobilisiert sind.

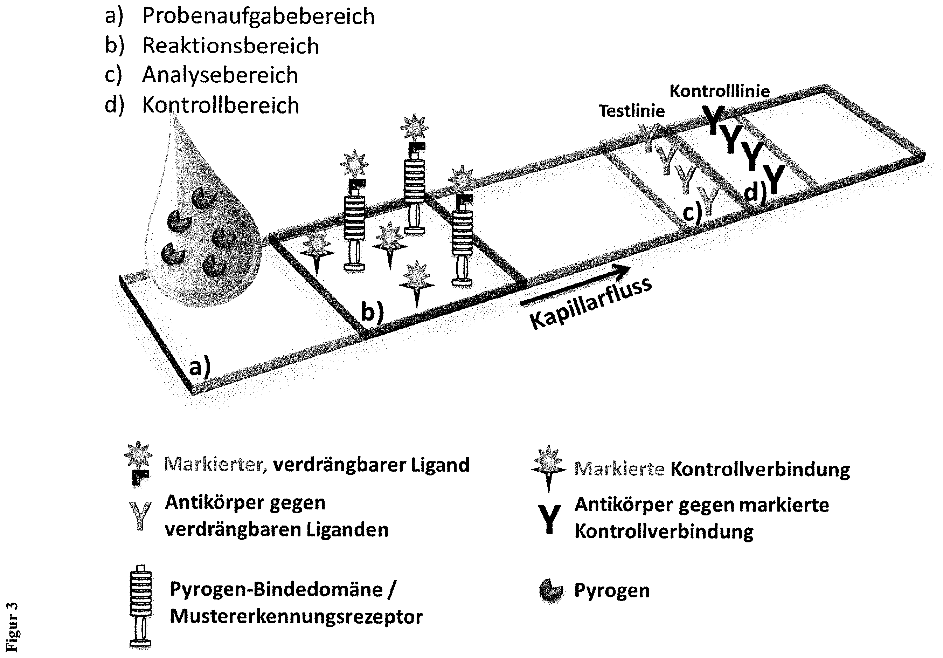

**Figur 3** zeigt das erfindungsgemäße Immunassay-Testsystem, wobei der Assayträger als kapillarflussfähiges Substrat ausgestaltet ist und im Reaktionsbereich b) Pyrogen-Bindedomänen eines PRRs immobilisiert und eine markierte Kontrollverbindung adsorbiert sind und eine Pyrogen-haltige Probe auf den Probeaufgabebereich aufgegeben wird.

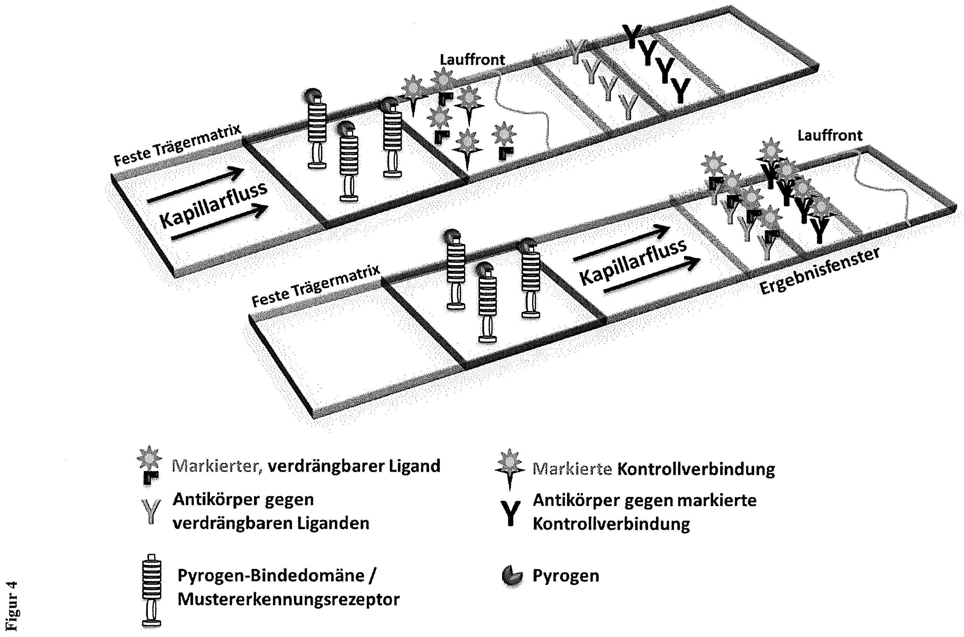

**Figur 4** zeigt die Verdrängung des markierten Liganden von der Pyrogen-Bindedomäne durch das Pyrogen. Gleichzeitig wird die adsorbierte Kontrollverbindung durch den Kapillarfluss mitgetragen. Im Ergebnisfenster wird der markierte Ligand von den Ligandenfängerproteinen (Antikörper) und die markierte Kontrollverbindung von den Kontrollverbindungsfängerproteinen (Antikörper) gebunden und so nachgewiesen.

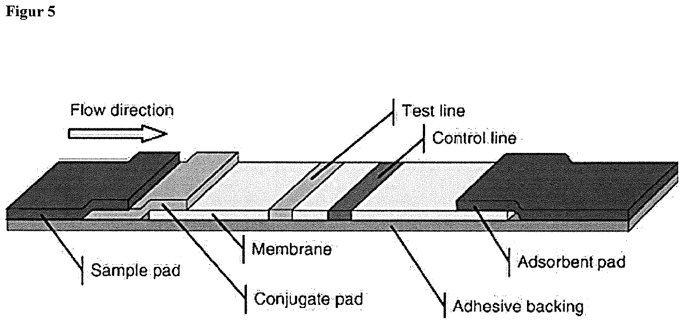

**Figur 5** zeigt einen weiteren möglichen schematischen Aufbau eines erfindungsgemäßen Assayträgers.

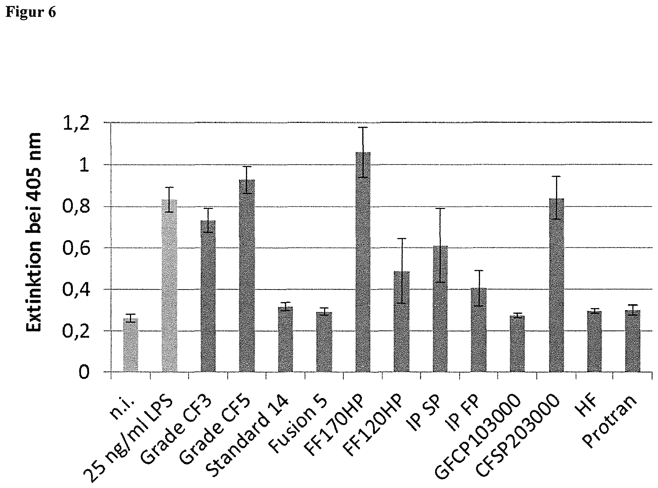

**Figur 6** zeigt die Ergebnisses eines PAMP-Assays. Dieser wurde mit der Reporterzelllinie NIH3T3 pNifty SEAP TLR4 / CD14 durchgeführt. 25 ng/mL LPS dienen als Positivkontrolle, n.i. ist die nicht induzierte Negativkontrolle. Der Substratumsatz (p-Nitrophenylphosphat) der im Überstand gebildeten SEAP wurde nach 60 min bei einer Wellenlänge von λ = 405 nm photometrisch ausgewertet.

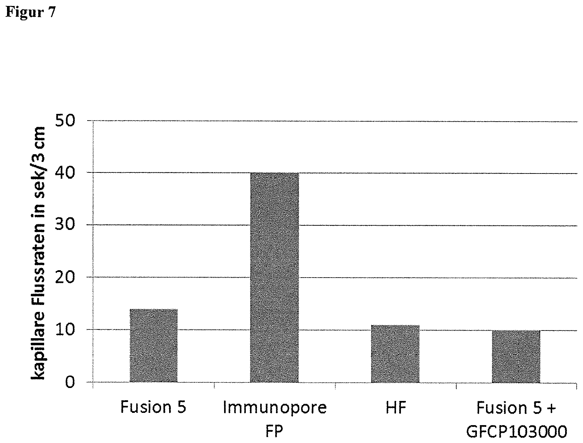

**Figur 7** zeigt die Flussraten der Membranen im Teststreifenaufbau mit 300 µL Probelösung. Aufbau der Teststreifen (Breite 2 cm): Standard 14 als Sample Pad (lcm Länge), verschiedene Membranen (5 cm Länge) und CF3 als Adsorbent Pad. Die Flussrate der Membranen Fusion 5, Immunopore FP, HF sowie Fusion 5 + Conjugate Pad GFCP10300 (1 cm Länge). Als Probelösung wurden 300 µL einer Ponceau S Lösung auf das Sample Pad aufgegeben.

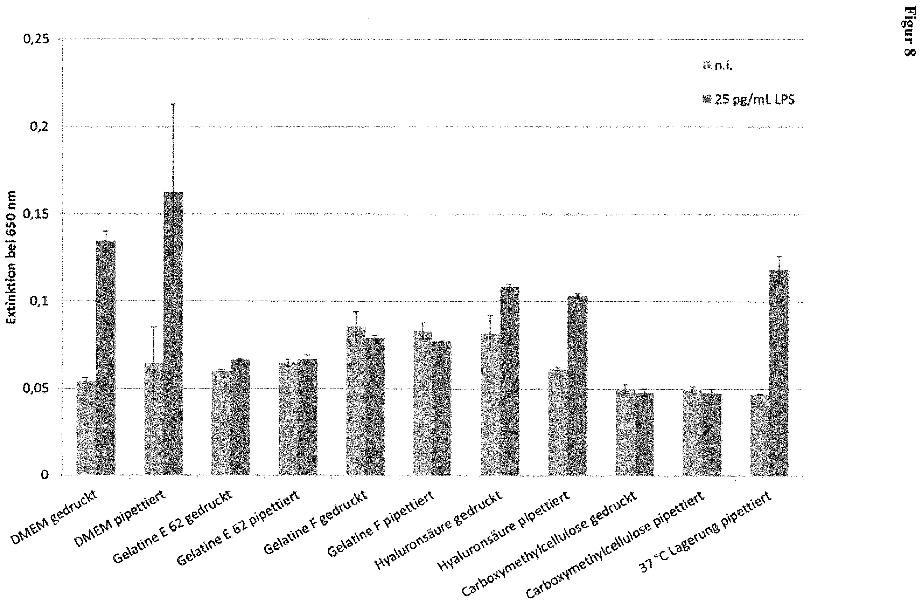

**Figur 8** zeigt die Untersuchung verschiedener Drucktinten. Hierfür wurden Zellen der Reporterzelllinie NIH3T3 pNifty SEAP TLR4 / CD14 in verschiedenen Drucktinten auf das Substrat gedruckt oder per Hand pipettiert und anschließend ein PAMP-Assay zur Überprüfung der Funktionalität der Rezeptoren durchgeführt. 25 ng/mL LPS dienten als Positivkontrolle, n.i. ist die nicht induzierte Negativkontrolle. Der Substratumsatz (Quanti-BlueTM, InvivoGen) der im Überstand gebildeten SEAP wurde nach 30 min bei einer Wellenlänge von λ = 650 nm.

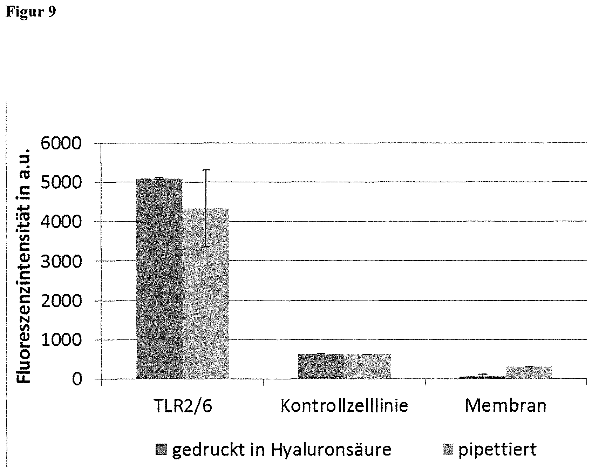

**Figur 9****:** Fluoreszenzintensitäten von an gedruckten Zellen gebundenen Pyrogene. NIH3T3 pNifty SEAP TLR2/6 Zellen und entsprechende Reporterzellen ohne Rezeptor wurden in einer Hyaluronsäure-Drucktinte auf Immunopore-Membranen gedruckt. Die Pyrogenbindung von Rhodamin-markiertem Pam3CSK4 wurde fluoreszenzphotometrisch bei λₑₓ = 549 nm und λₑₘ = 566 nm gemessen. Zur Kontrolle des Druckprozesses wurden die Zellen auch von Hand auf die Membran pipettiert.

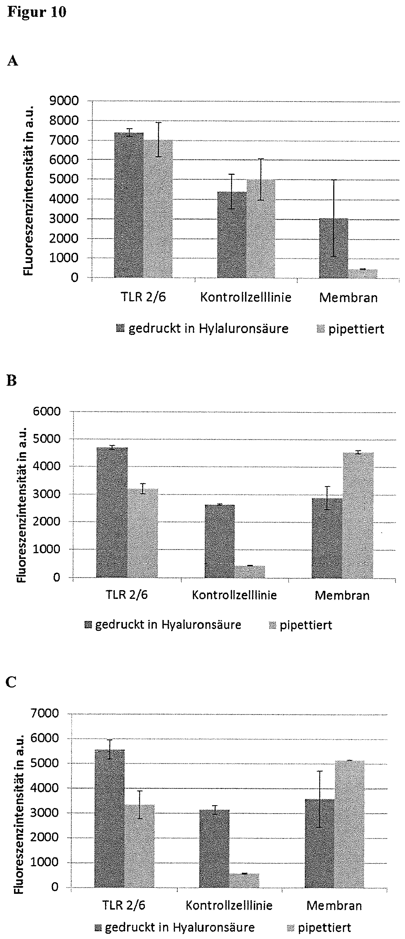

**Figur 10** Fluoreszenzintensitäten von an gedruckten und konservierten Zellen gebundenen Pyrogenen. NIH3T3 pNifty SEAP TLR2/6 Zellen und entsprechende Reporterzellen ohne Rezeptor wurden in einer Hyaluronsäure-Drucktinte auf Immunopore-Membranen gedruckt und unterschiedlich konserviert. Die Pyrogenbindung von Rhodamin-markiertem Pam3CSK4 wurde fluoreszenzphotometrisch bei λₑₓ = 549 nm und λₑₘ = 566 nm gemessen. Zur Kontrolle des Druckprozesses wurden die Zellen auch von Hand auf die Membran pipettiert. Konservierungsmethoden: A) bei 37°C über Nacht getrocknet; B) mit 70 % Ethanol fixiert; C) für 96 h bei 4°C gelagert.

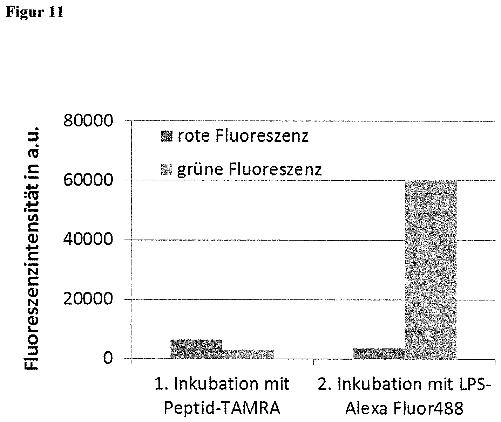

**Figur 11** Verdrängung eines verdrängbaren Liganden durch LPS. Auf Ni-NTA Magnetic Agarose Beads (Qiagen), an denen TLR4 über das His-Tag immobilisiert wurde, wurde zunächst das TAMRA-markierte Peptid GLLHIFYIPRRDLKQLYFNL als verdrängbarer Ligand an TLR4 gebunden (1. Inkubation). In der nachfolgenden zweiten Inkubation wurde gebundenes Peptid durch Alexa Fluor 488-markiertes LPS verdrängt. Die Verdrängung wurde mittels Fluoreszenzmikroskopie und fluoreszenzphotometrischer Auswertung verfolgt (2. Inkubation).

### Beispiel 1: Nachweis von LPS durch TLR4

### 1.1 Materialauswahl

Pyrogene, Bakterien und ihre Überreste, kommen überall in unserem Alltag und in Produktionsprozessen vor und können nur durch trockene Hitze von mehr als 180°C inaktiviert werden. Damit ist die absolute Pyrogenfreiheit aller verwendeten Materialien essentiell (Einzelkomponenten des Immunassay-Testsystems, Lösungen, Puffer, Substrate, Laborverbrauchsmaterialien, Laborgeräte). Dies ist notwendig, da Pyrogene eine natürlich hohe Affinität zu dem hier als Sensorelement verwendeten TLR4, also der Pyrogen-Bindedomäne, besitzen, an diesen binden und somit die Funktionsweise des Immunassay-Testsystems nachteilig beeinträchtigen oder gänzlich verhindern.

Mit Hilfe eines etablierten PAMP-Assays (DE 10 2006 031 483; EP 2 041 172) wurden konsequent alle Komponenten des Immunassay-Testsystems wie Substrate, Blocklösungen, etc. auf ihre Pyrogenfreiheit untersucht. Für diesen Assay wurde der Rezeptorkomplex TLR4 in eine NIH3T3-Fibroblastenzellinie stabil transfiziert und exprimiert sowie eine sekretierte, alkalische Phosphatase (SEAP) als Reportergen stabil integriert. Die Aktivierung von TLR4 durch Pyrogene führt zur Aktivierung des Transkriptionsfaktors NF-_{K}B, der wiederum die SEAP-Expression induziert. Pyrogene, welche sich in oder auf potentiellen Komponenten des Immunassay-Testsystems befinden, können so durch die Expression des Reportergens nachgewiesen werden und anschließend nur pyrogenfreie Komponenten zum Aufbau des Immunassay-Testsystems ausgewählt werden.

Figur 5 zeigt beispielhaft den möglichen Aufbau eines erfindungsgemäßen Assayträgers, der als kapillarflussfähiges Substrat ausgestaltet ist.

Der Assayträger eines erfindungsgemäßen Teststreifens, das heißt vorliegend das kapillarflussfähige Substrat, setzt sich im Regelfall aus einem Verbund mehrerer Materialien zusammen, die jeweils einzeln eine spezifische Aufgabe übernehmen. Figur 5 zeigt schematisch einen solchen Aufbau aus Sample Pad, Conjugate Pad, Membran und Adsorbent Pad.

Die zu analysierende Probe wird im Bereich des Sample Pads, das heißt dem Probeaufgabebereich, aufgegeben. Dieser gewährleistet einerseits einen gleichmäßigen und homogenen Probentransport und kann andererseits zum Rückhalt von Partikeln eingesetzt werden. Auf dem überlappenden Conjugate Pad, das heißt dem Reaktionsbereich, werden TLR4-Pyrogenbindedomänen immobilisiert. Dies kann direkt auf dem Material erfolgen oder mit Hilfe von Partikeln wie Agarosebeads, die hier vor Ort gehalten werden. Zusätzlich kann das Conjugate Pad Pufferchemikalien, Blockreagenzien oder Stabilisatoren enthalten. Über die Membran erfolgt die Auftrennung zwischen Kontrollverbindung und dem verdrängbaren TLR4 Liganden als Detektionsmolekül. An ihrem Ende werden beide Molekülarten über Ligandenfängerproteine bzw. Kontrollverbindungsfängerproteine zum Anzeigen des Testergebnisses gefangen. Das Adsorbent Pad (adsorbierendes Material) am Ende des Teststreifens nimmt überschüssige Flüssigkeit auf und gewährleistet den notwendigen Kapillarfluss im Teststreifen.

**Tabelle 1: Zusammenstellung der untersuchten Materialien für das Substrat**

| **Funktion** | **Bezeichnung** | **Bezugsquelle** | **Technische Daten** |
|---|---|---|---|
| **Sample Pad** | CF3 | GE Healthcare | Sample und Adsorption Pad, Baumwoll-Linter, 322 µm Dicke |
| **Sample Pad** / **Adsorbent pad** | CFSP203000 | Merck Millipore | Zellulose,83 µm Dicke, kann sowohl als Sample als auch als Adsorbent Pad verwendet werden |
| **Conjugate Release Pad** | Standard 14 | GE Healthcare | Glasfaser, 355 µm Dicke |
| **Conjugate Release Pad** | GFCP103000 | Merck Millipore | Glasfaser, 48 µm Dicke |
| **Membran** | FF120HP | GE Healthcare | Nitrozellulosemembran (100µm), mit Plastikrücken (100µm), Flussrate: 90-150 s/4cm |
| **Membran** | FF170HP | GE Healthcare | Nitrozellulosemembran (100µm), niedrige Viskosität, mit Plastikrücken (100µm), Flussrate: 140-200 s/4cm |
| **Membran** | Immunopore SP | GE Healthcare | Nitrozellulosemembran (100µm), mit Plastikrücken (100µm), Flussrate: 190-280 s/4cm |
| **Membran** | Immunopore FP | GE Healthcare | Nitrozellulosemembran (100µm), mit Plastikrücken (100µm), Flussrate: 140-200 s/4cm |
| **Membran** | Millipore High Flow Plus | Merck Millipore | Nitrocellulose |
| **Membran** | Protran BA85 | Whatman | Nitrocellulose, Porengröße: 0,45 µm |
| **Adsorbent Pad** | CF3 | GE Healthcare | Sample und Adsorption Pad, Baumwoll-Linter, 322 µm Dicke |
| **Adsorbent Pad** | CF5 | GE Healthcare | Absorption Pad, Baumwoll-Linter, 954 µm Dicke |
| **Sondermaterial** | Fusion 5 | GE Healthcare | Glasfaser mit Kunststoffrückteil, Material, das zeitgleich alle oben genannten Funktionen übernimmt |

Die untersuchten Substrate (siehe Tabelle 1) wurden von GE Healthcare, Merck, Millipore und Whatman bezogen. Sie alle werden jedoch nicht unter sterilen Bedingungen produziert, was in den im PAMP-Assay untersuchten Materialeluaten gezeigt werden konnte (Figur 6). Insbesondere die Adsorbent Pads CF3, CF5 und CFSP203000 zeigen eine sehr starke Aktivierung der TLR4 Rezeptoren und damit eine Pyrogenverunreinigung. Ein Einsatz dieser Materialien für den Aufbau des Teststreifens ist dennoch möglich, da sie nicht in direktem Kontakt mit dem Reaktionsbereich am gegenüberliegenden Ende des Teststreifens in Kontakt kommen. Lediglich auf den Einsatz des Materials CFSP203000 als Sample Pad sowie der Einsatz der Membran FF170 HP wurde verzichtet. Für die Materialien des sensibelsten Reaktionsbereichs, dem Conjugate Pad, war keine Aktivierung der TLR4 nachzuweisen. Damit eignen sich die untersuchten Materialien im Wesentlichen zum Aufbau des erfindungsgemäßen Immunassay-Testsystems.

Ein wichtiger Parameter des Immunassay-Testsystems ist die Flussrate der Flüssigkeitsfront im Teststreifen, denn sie bestimmt die Kontaktzeit mit der Pyrogenbindedomäne des TLR4-Rezeptors und damit die Zeit, die zur Verdrängung des farbmarkierten, verdrängbaren Liganden durch LPS, zur Verfügung steht. Daher wurden zur weiteren Charakterisierung der Substrate exemplarisch Teststreifen mit einer Breite von 2 cm nach dem Schema in Figur 5 aufgebaut. Alle Teststreifen bestanden aus Standard 14 als Sample Pad (1 cm Länge) und CF3 als Adsorbent Pad (2 cm Länge). Die Flussrate der Membranen Fusion 5, Immunopore FP, HF sowie Fusion 5 + Conjugate Pad GFCP10300 (1 cm Länge), jeweils mit einer Länge von 5 cm wurden wie folgt analysiert: Unterschiedliche Volumina (100 / 200 / 300 / 400 / 500 µL) einer Ponceau S Lösung wurden auf das Sample Pad aufgegeben. Es wurde die Gleichmäßigkeit der Lauffront, die Saugfähigkeit der Materialien und damit das benötigte Mindestvolumen der Probe, sowie die Zeit, die die Lauffront bis zum Erreichen einer definierten Laufstrecke von 3 cm benötigt, bewertet. Figur 7 stellt die Flussraten der unterschiedlichen Membranen bei Aufgabe von 300 µL Ponceau S Lösung dar.

Die Membran Immunopore FP hebt sich von den anderen 3 untersuchten Membranen deutlich ab, da sie kaum saugfähig ist und mit 40 s/3 cm die höchste Flussrate bei einer gleichmäßigen Lauffront besitzt. Insbesondere das Material Fusion 5 ist ein sehr saugstarkes Material, das durch größere Probenvolumina bis zu 500 µL gut aufnehmen kann, hingegen bei zu geringen Probenvolumen nicht funktionsfähig ist. Durch zusätzliche Kombination der Fusion 5 mit einem Conjugate Pad (GFCP103000) wird die Saugfähigkeit weiter erhöht und die Flussrate sinkt auf 10 sek/ 3 cm. Es stehen also für verschiedene Anforderungen hinsichtlich, Probenvolumina und Testschnelligkeit unterschiedliche Materialien zur Verfügung.

Um unspezifische Bindungen der markierten Kontrollverbindungen und Liganden auf den untersuchten Membranen zu vermeiden, müssen die nicht besetzten Membranbindungsstellen abgesättigt, das heißt blockiert, werden. Am geeignetsten hinsichtlich Pyrogenfreiheit, Reproduzierbarkeit und Zelltoxischer Wirkung zeigte sich Roti® Block (Carl Roth GmbH).

### 1.2 Bereitstellung des Rezeptors

Isolierte TLR4-Rezeptoren oder deren Pyrogenbindedomänen wurden für das Immunassay-Testsystem über folgende Quellen bezogen:
- Rekombinantes TLR4 aus NS0-Zellen (Mausmyelomazellen), R&D Systems, 3146-TM-050/CF
- TLR4 Proteinfragment aus Weizenkeimlysat, Abcam, ab1112362
- TLR4 / CD284 aus Baculovirus-Insektenzellen, Hölzel Diagnostik GmbH, 10146-H08B-100

Insbesondere wurde im Folgenden TLR4 / CD284 (Hölzel Diagnostik GmbH) verwendet.

### 1.3 Fluoreszenzmarkierung des verdrängbaren Liganden und der Kontrollverbindung

Zum Nachweis der Verdrängung des verdrängbaren Liganden und zum Nachweis der Funktionsfähigkeit des Teststreifens über die Kontrollverbindung müssen diese, nachdem sie von entsprechenden Fängerproteinen gebunden wurden, detektiert werden. Mittels Farbmarkierungen sind entsprechende Moleküle besonders einfach zu detektieren. Die Methode der Fluoreszenzmarkierung, der Markierung mit Fluorophoren, ist hierbei besonders im Vergleich zur visuellen Auswertung nicht-fluoreszierender Farbstoffe vorteilhaft, da bereits geringe Mengen Fluorophor mit entsprechenden Detektoren auch unterhalb der visuellen Detektionsgrenze nachgewiesen werden können. Ein weiterer Vorteil der Fluoreszenzmarkierung besteht in der Möglichkeit zur parallelen Messung verschiedenfarbiger Fluorophore.

Besonders geeignete Fluorophore sind durch eine starke Absorption (einen hohen Extinktionskoeffizienten ε), eine hohe Quantenausbeute (Q > 0,7) und einen großen Stokes-Shift gekennzeichnet. Vorliegend wurde TAMRA als Farblabel für den verdrängbaren Liganden und die Kontrollverbindung ausgewählt, da es einen großen Extinktionskoeffizienten von 95.000 L cm⁻¹ mol⁻¹ besitzt und zudem gut visuell sichtbar ist.

### 1.4 Bereitstellung des verdrängbaren Liganden

Mit Hilfe des bereits beschriebenen PAMP-Assays wurden verschiedene small molecules (kleine Moleküle) und Peptide als verdrängbare Liganden identifiziert. Das nachzuweisende Pyrogen E. coli LPS besitzt im PAMP-Assay mit der Reporterzelllinie NIH3T3 pNifty SEAP TLR4 / CD14 eine mittlere effektive Konzentration (EC50) von 40 pg/mL. Damit LPS einen am Rezeptor oder dessen Pyrogenbindedomäne gebundenen Liganden verdrängen kann, muss dieser eine geringere Bindungsaffinität besitzen, was mit einem geringeren EC50-Wert korreliert. Mit Hilfe des PAMP-Assays wurden vier Peptide und zwei small molecules als verdrängbare Liganden mit agonistischer Wirkung identifiziert:
- small molecule T5635346 (2-Hydroxy-4-methylphenyl)-morpholin-4-ylmethanone); IC50 von 7,7 mM
- small molecule T6854457 (4-Methyl-N-(2-oxoazepan-3-yl)benzenesulfonamide); IC50 von 2,6 mM
- Peptid "KGETVNTTISFSFKGIKFSK"; IC50 > 200 mM, (SEQ ID Nr. 1)
- Peptid "KMQYPISINVNPCIELKGSK"; IC50 > 200 mM, (SEQ ID Nr. 2)
- Peptid "GLLHIFYIPRRDLKQLYFNL"; IC50 > 200 mM, (SEQ ID Nr. 3)
- Peptid "KRKEVICRGSDDDYSFCRAL"; IC50 > 200 mM, (SEQ ID Nr. 4)

Die Affiniät eines Liganden zu seinem Rezeptor beschreibt seine Neigung mit dem Rezeptor eine Bindung einzugehen. Je höher die Affinität, desto größer ist die Assoziationskonstante Kₐ. Die Stärke der Rezeptor-Ligand-Weclvselwirkung lässt sich experimentell mit Hilfe der Oberflächenplasmonenresonanzspektroskopie (Biacore T200, GE Healthcare) bestimmen und durch die sogenannte Dissoziationskonstante (K_{d}) quantifizieren. Dabei ist K_{d}, der reziproke Wert von Kₐ, eine thermodynamische Größe, die angibt, welcher Anteil des Liganden im Mittel an das Rezeptorprotein gebunden ist und hat die Dimension einer Konzentration. Das Biacore-Chipsystem basiert auf einem Glasträger mit einem Goldfilm in einer Flusszelle. TLR4 wurde auf dem NTA-Sensorchip über sein His-Tag nach Herstellerprotokoll immobilisiert und die zu untersuchenden Liganden, Kontrollverbindungen und LPS wurden gelöst in HBS-P+ mit 50 µM EDTA bei 20 °C mit einer Flussrate von 10 µL/min in jeweils 5 Konzentrationen darüber geleitet. Die Bindung mit TLR4 wird als Änderung des Brechungsindex in der Flusszelle im Vergleich zu einer Referenzzelle als response difference in RU gemessen. Dabei entspricht 1 RU 1 pg gebundenem Ligand. Nach Ligandeninjektion wird mit Puffer nachgespült, um idealerweise den Ausgangszustand, vollständige Dissoziation des Liganden, wieder herzustellen.

Tabelle 2 listet die mit Hilfe der Oberflächenplasmonenresonanzspektroskopie ermittelten Bindungseigenschaften des small molcules T5635346 mit und ohne TAMRA-Markierung sowie von mit Alexa Fluor 488 markiertem LPS von *Salmonella minnesota*. T5635346 besitzt eine Affinität zu TLR4 in der gleichen Größenordnung wie das hier untersuchte LPS. Durch das TAMRA-Label verringerte sich jedoch die Bindungsaffinität um das 40-fache.

**Tabelle 2: Bindungseigenschaften des small molcules T5635346 zu TLR4**

| **Ligand** | **MW in g/mol** | **K_{d} in M** | **Kₐ in 1/M** |
|---|---|---|---|
| **T5635346** | 221 | 2,42E-07 | 4.135.649 |
| **T5635346-TAMRA** | 723 | 8,50E-06 | 117.633 |
| **LPS *S. minnesota* Alexa Fluor 488** | | 2,83E-07 | 4.203.447 |

### 1.5 Bereitstellung der Kontrollverbindung

Analog zur Identifizierung der verdrängbaren Liganden wurden auch nicht an den Rezeptor bindende small molecules und Peptide als Kontrollverbindungen identifiziert:
- small molecule T5874283 (1-(1,3-benzodioxol-5-yl)-3-[[4-(2-methylpropyl)morpholin-2-yl]methyl]urea)
- small molecule T7054880 (1-[1-(3-methoxyphenyl)propan-2-yl]-3-(1H-pyrazot-4-yl)urea)
- Peptid "FMMLGGLVRI", (SEQ ID Nr. 5)
- Peptid "RMMWFGIMV", (SEQ ID Nr. 6)
- Peptid "SGSPEEMLFCLEFVILHQPNSN", (SEQ ID Nr. 7)

Mit Hilfe der Oberflächenplasmonenresonanzspektroskopie, siehe Abschnitt 1.4, wurde nachgewiesen dass die als Kontrollverbindungen ausgewählten small molecules T7054880 und T5874283 nicht oder mit einer ähnlichen Affinität wie LPS an TLR4 binden (siehe Tabelle 3).

**Tabelle 3: Bindungseigenschaften der Kontrollverbindungen zu TLR4**

| **Ligand** | **MW in g/mol** | **K_{d} in M** | **Kₐ in 1/M** |
|---|---|---|---|
| T5874283 | 335 | 1,02E-07 | 9.803.922 |
| T7054880 | 274 | - | - |

### 1.6 Immobilisierung des Rezeptors auf dem Substrat

Neben der Immobilisierung isolierter Rezeptoren (siehe Abschnitt 1.2) wurden weiterhin Zellen der Reporterzelllinie auf die Nitrocellulosemembran Immunopore FP mit Hilfe eines Table-top robot mta TR300 Druckers (mta automation Inc.) gedruckt. Hierfür wurden zunächst geeignete zellverträgliche Drucktinten, wie z. B. Tinten basierend auf Hyaluronsäure (Lifecore Biomedicals), Gelatine (Gelita AG) bzw. Carboxymethylcellulose (Sigma-Aldrich GmbH), untersucht. Dazu wurden jeweils 100 µl mit einer absoluten Zellzahl von 30.000 Zellen je Well einer 96-Well Microtiterplatte bei einer Geschwindigkeit von 15 µl/s gedruckt oder als Druckkontrolle von Hand pipettiert und die Zellen über Nacht bei 37°C inkubiert. Am zweiten Tag wurden die Zellen mit 25 ng/mL LPS induziert und am dritten Tag wurde die sekretierte alkalische Phosphatase im Überstand durch den Umsatz des Substrates Quanti-BlueTM (InvivoGen) detektiert (Figur 8).

Anhand des Vergleichs der in DMEM gedruckten und von Hand pipettierten Zellen wurde gezeigt, dass der Druckprozess im Vergleich zum von Hand Pipettieren nur geringe Auswirkungen auf die Zellen hat. Von den untersuchten Druckmedien wurde eine Drucktinte aus 1 % Hyaluronsäure gelöst in DMEM Medium als geeignet identifiziert und für die folgenden Experimente verwendet.

Zur Immobilisierung ganzzelliger Reporterzellen auf Membranen wurden alternativ zu TLR4 exprimierenden Zellen NIH3T3 pNifty SEAP TLR2/6 auf die Nitrocellulosemembran Immunopore gedruckt. Ebenso wurde eine korrespondierende Reporterzelllinie ohne TLR4 zur Kontrolle einer unspezifischen Bindung des Pyrogens verwendet. Hierzu wurden je vier Punkte mit je 40.000 Zellen pro Versuchsbedingung mit einer Geschwindigkeit von 15 µl/s auf Nitrocellulosemembran Immunopore FP (1,5 cm x 1,5 cm) gedruckt. Als Kontrolle wurden ebenfalls 40.000 Zelle in 100 µL Medium in je vier auf die Membran aufgesetzten Klonringen von Hand pipettiert, um sie ortsfest adhärieren zu lassen und dem Druckmuster ähnliche Versuchsbedingungen für die Auswertung zu gewährleisten. Nach einer Inkubation bei 37°C über Nacht wurde am darauffolgenden Tag das für NIH3T3 pNifty SEAP TLR2/6 spezifische Pyrogen Pam3CSK4 mit Rhodamin-Markierung (6 ng/mL, Invivogen) zugegeben und für 4 h zur Bindung am Rezeptor bei 37°C inkubiert. Vor der fluoreszenzphotometrischen Messung des gebundenen Pam3CSK4 mit Rhodamin-Markierung λₑₓ = 549 nm und λₑₘ = 566 nm wurden die einzelnen Proben mit PBS gewaschen, um nicht gebundenes Pyrogen zu entfernen. Figur 9 zeigt die Ergebnisse des oben beschriebenen Versuchs zur Immobilisierung ganzzelliger Reporterzellen auf Membranen mittels Druckverfahren. Die Vitalität der gedruckten Zellen war für diesen Versuchsaufbau von untergeordneter Relevanz. Hauptfokus lag darauf, dass die Membranrezeptoren beim Druckprozess nicht beschädigt wurden und weiter die Fähigkeit zur Pyrogenbindung besitzen. Es wurde gezeigt, dass durch das Immobilisieren mittels Druckverfahren die Membranrezeptoren im direkten Vergleich zu von Hand pipettierten Zellen nicht beschädigt werden. Zudem wurde gezeigt, dass keine unerwünschte unspezifische Bindungen zwischen Pyrogen und Membran oder Pyrogen und Druckmedium auftritt. Das Drucken bietet somit den Vorteil, dass ganzzellige Sensorelemente auf einem Substrat genau ortsaufgelöst positioniert werden können.

Neben dem Aufbau eines Teststreifens ist aber auch seine Lagerfähigkeit zu betrachten. Werden ganzzellige Sensorelemente, also Rezeptorzellen, auf eine Oberfläche ohne Schutzschicht aufgebracht besteht die Gefahr, dass Membranrezeptoren bei der überführung der Zellen in einen lagerfähigen Zustand beschädigt werden und ihre Funktionalität einbüßen. Auch für diese Problematik kann das Drucken der Zellen eine Lösung sein, da die Zellen hier in einer gelartigen Drucktinte abgelegt werden, die Zellen vor äußeren Einflüssen schützen. Hierfür wurden ganzzellige Sensorelemente wie weiter oben beschrieben verdruckt und zur Kontrolle von Hand pipettiert. Entsprechende Proben wurden dreierlei Konservierungs- und Lagermethoden unterzogen: Trocknung bei 37°C für 24 h, Fixierung mit 70 % Ethanol und Lagerung bei 4°C für 96 h. Nach Konservierung und Lagerung wurde die Bindung des spezifischen Pyrogens Pam3CSK4 mit Rhodamin-Markierung wie bereits oben beschrieben untersucht.

Figur 10 zeigt die Ergebnisse dieser Untersuchungen. Im Vergleich zu den Ergebnissen in Figur 9 wurden stets höhere Werte der Pyrogenbindung an die Kontrollzellline und die Membran nachgewiesen. Hierbei handelt es sich um unspezifisch gebundenes Pyrogen. Zur Reduzierung der unspezifischen Pyrogenbindung an der Membran kann diese mittels Roti-Block geblockt werden. In Figur 10A sind die Ergebnisse nach der Trocknung dargestellt, in 10B die Ergebnisse der Ethanolfixierung und in 10C die Ergebnisse nach Lagerung bei 4 °C. Bei einem hohen Background durch unspezifische Pyrogenbindung an Membran und Kontrollzelllinie wurde dennoch bei allen Konservierungs- und Lagermethoden ein eindeutiges Delta der Fluorszenzintensität von mehr als 3000 a.u. nachgewiesen. Der Vergleich von gedruckten zu den von Hand pipettierten Rezeptorzellen, die nicht in einer gelartigen Schutzmatrix eingebettet sind, zeigte, dass die umgebende Schutzschicht einen Funktionsverlust der TLRs bei Lagerung und Konservierung verhindert.

### 1.7 Antikörper als spezifische Ligandenfängerproteine

Spezifische Fängerproteine binden ortsaufgelöst den verdrängbaren Liganden bzw. die Kontrollverbindung. Hierfür eignen sich besonders Antikörper. Diese können entweder spezifisch den verdrängbaren Liganden bzw. die Kontrollverbindung oder spezifisch gegen das Fluorophor gerichtet sein, welches zur Farbmarkierung der Moleküle verwendet wird. In Abschnitt 1.3 wurden die Fluoreszenzfarbstoffe Tetramethyl-rhodamine 5-Carboxamido-(6-Azidohexanyl) (TAMRA) und Alexa Fluor 488 zur Markierung von Kontrollverbindungen und verdrängbaren Liganden als geeignet identifiziert. Gegen diese Fluorophore sind spezifische Antikörper kommerziell erhältlich, so dass eine individuelle Antikörperherstellung für ausgewählte verdrängbare Liganden bzw. Kontrollverbindungen gegebenenfalls entfallen kann, wenn die Farbeigenschaften des Fluorophors bei Bindung an das Antigen nicht verloren gehen.

Die Auswirkung der Antikörperbindung auf die Fluorophore Alexa Fluor 488 und TAMRA wurde mittels spezifischem Dot Blot untersucht. Hierzu wurden zwei verschiedene Konzentrationen der Farbstoffe (100 und 500 ng für Alexa Fluor 488 sowie 7 und 35 ng für TAMRA) auf eine Nitrocellulosemembran gedottet und weiter mit den spezifischen Antikörpern inkubiert. Die Bindung der Antikörper an das jeweilige Fluorophor wurde durch die am Antikörper konjugierte Meerrettichperoxidase immunodetektiert. Es wurde für beide Fluorophore mit Hilfe der Fluoreszenzmikroskopie gezeigt, dass die Antikörper spezifisch an ihr jeweiliges Antigen binden und diese dabei keine Einbußen in der Fluoreszenzintensität aufweisen. Damit eignen sich die ausgewählten anti-TAMRA und anti-Alexa Fluor 488 Antikörper als Fängerproteine zum Aufbau des Imunassay-Testsystems.

### 1.8 Verdrängbarer Ligand-Rezeptor-Komplex

Das zentrale Assayselement des Imunassay-Testsystems besteht aus dem verdrängbaren Liganden-Rezeptor-Komplex. Dieser wird bei Anwesenheit von Pyrogenen aufgebrochen, wobei das Pyrogen den verdrängbaren Liganden verdrängt, an den Rezeptor bindet und der verdrängbare Ligand freigesetzt wird. Dies wurde mit Hilfe von Ni-NTA Magnetic Agarose Beads (Qiagen), an die TLR4 (Hölzel Diagnistik GmbH) mittels His-Tag gebunden wurde, gezeigt (siehe Figur 11).

In einem ersten Schritt wurden mit TLR4 bestückte Magnetic Agarose beads mit TAMRA-markiertem Peptid GLLHIFYIPRRDLKQLYFNL bzw. mit TAMRA-markiertem small molecule T5635346 als verdrängbare Liganden für 1 h bei Raumtemperatur in Bindepuffer (50 mM NaH2PO2*2H2O; 300 mM NaCl; 20 mM Imidazol, pH 8,0) inkubiert. Nicht gebundene Liganden wurden in einem Waschschritt entfernt. Im zweiten Schritt wurden die Magnetic Agarose beads mit dem TLR4-verdrängbarer Ligandenkomplex mit Alexa Fluor 488-markiertem *Salmonella minnesota-*LPS in Interaction Buffer (Bindepuffer + 0,05% Tween20) inkubiert. Nicht gebundene und ggf. verdrängte Liganden wurden im anschließenden Waschschritt entfernt. Nach jeder Inkubation wurden Fluoreszenzaufnahmen der Magnetic Agarose beads angefertigt. Nach dem ersten Inkubationsschritt ist z. B. die Bindung des Peptids, also des verdrängbaren Liganden, über die TAMRA-Markierung auf der Beadoberfläche als rote Färbung nachweisbar. Nach dem zweiten Inkubationsschritt ist die Verdrängung und Freisetzung des bisher gebundenen Peptids durch die Bindung von LPS über die Alexa Fluor 488-Markierung auf der Beadoberfläche als grüne Färbung nachweisbar. Figur 11 zeigt die zugehörige fluoreszenzphotometrische Auswertung. Von der ersten zur zweiten Inkubation verringert sich die gemessene Fluoreszenz von TAMRA. Dies bedeutet, dass der TAMRA-markierte Ligand verdrängt wurde. Gleichzeitig erhöht sich jedoch die Fluoreszenz von Alexa Fluor 488 von der ersten zur zweiten Inkubation. Dies bedeutet wiederum, dass das Alexa Fluor 488-markierte Pyrogen LPS die bisherigen Bindestellen des TAMRAmarkierten verdrängbaren Liganden eingenommen hat.

## Patentansprüche

1. Kompetitives Immunassay-Testsystem zum Nachweis mindestens eines in einer flüssigen Probe enthaltenen Pyrogens, umfassend einen Assayträger, aufweisend mindestens eine immobilisierte Pyrogen-Bindedomäne mindestens eines Mustererkennungsrezeptors (PRRs, pattern recognition receptor) mit mindestens einem markierten, verdrängbaren Liganden, wobei die Verdrängung des markierten Liganden von der Pyrogen-Bindedomäne des PRRs durch das in der Probe enthaltene Pyrogen durch eine Farbreaktion der Markierung angezeigt wird und wobei der Assayträger mindestens ein immobilisiertes Ligandenfängerprotein zur Bindung des verdrängten, markierten Liganden aufweist und die Bindung des Liganden an das Ligandenfängerprotein durch eine Farbreaktion der Markierung angezeigt wird.

2. Das Testsystem gemäß Anspruch 1, wobei der markierte, verdrängbare Ligand und die mindestens eine immobilisierte Pyrogen-Bindedomäne so ausgestaltet sind, dass die Farbreaktion bei Verdrängung des markierten Liganden von der Pyrogen-Bindedomäne stattfindet.

3. Das Testsystem gemäß einem der Ansprüche 1 bis 2, wobei der Assayträger eine Mikrotiterplattenvertiefung, ein Reagenzgefäß, ein Teströhrchen oder ein kapillarflussfähiges Substrat ist.

4. Das Testsystem gemäß Anspruch 3, wobei das kapillarflussfähige Substrat in Kapillarflussrichtung folgende, jeweils voneinander beabstandete Bereiche aufweist:
a) einen Probeaufgabebereich,
b) einen Reaktionsbereich, aufweisend die mindestens eine immobilisierte Pyrogen-Bindedomäne mindestens eines PRRs mit dem mindestens einen markierten, verdrängbaren Liganden und
c) einen Analysebereich, aufweisend das mindestens eine immobilisierte Ligandenfängerprotein zur Bindung des verdrängten, markierten Liganden.

5. Das Testsystem gemäß einem der Ansprüche 3 oder 4, weiterhin umfassend
b') einen Kontrollverbindungsbereich, aufweisend mindestens eine adsorbierte, markierte Kontrollverbindung, die nicht an die Pyrogen-Bindedomäne des PRRs oder das Ligandenfängerprotein bindet und
d) einen Kontrollbereich, aufweisend mindestens ein immobilisiertes Kontrollverbindungsfängerprotein zur Bindung der mindestens einen, markierten Kontrollverbindung.

6. Das Testsystem gemäß einem der Ansprüche 3 bis 5, wobei die Bereiche a), b), c) und d) jeweils voneinander beabstandet sind und die Bereiche b) und b') miteinander überlappen oder voneinander beabstandet sind.

7. Das Testsystem gemäß einem der vorstehenden Ansprüche, wobei der verdrängbare Ligand und, falls vorhanden, die Kontrollverbindung mit der gleichen oder unterschiedlichen Farbmarkierung(en) ausgewählt aus der Gruppe bestehend aus Fluoreszenzfarbstoffen, insbesondere Fluoresceinsothiocyanat (FITC), Fluorescein, Rhodamin, ATTO-Farbstoffen, Texas Red, Phycoerythrin-Derivaten, Cyanin-Fluoreszenzfarbstoffen; farbigen Latexpartikeln, Rußpartikeln, magnetischen Partikeln, Quantum dots, kolloidalen Selenpartikeln, Goldnanopartikeln, Goldclustern, kolloidalen Goldpartikeln; Enzymen, insbesondere Meerrettich-Peroxidase (HRP), Alkalische Phosphatase (AP), Glucoseoxidase (GOx), β-D-Galactosidase (β-Gal), Glucose 6-Phosphat Dehydrogenase (G6PD); chemilumineszenten Verbindungen, insbesondere Acridiniumester, Acridiniumsulfonamid, Isoluminol; Streptavidin, Avidin und Biotin markiert sind.

8. Das Testsystem gemäß einem der vorstehenden Ansprüche, wobei die flüssige Probe eine klinische Probe, eine medizintechnische Probe, eine pharmazeutische Probe, eine lebensmitteltechnologische Probe oder eine Umweltprobe ist.

9. Das Testsystem gemäß einem der vorstehenden Ansprüche, wobei die klinische Probe menschlichen oder tierischen Ursprungs ist.

10. Das Testsystem gemäß einem der vorstehenden Ansprüche, wobei die mindestens eine, immobilisierte Pyrogen-Bindedomäne ausgewählt ist aus der Gruppe bestehend aus den Pyrogen-Bindedomänen eines Toll-like-Rezeptors (TLRs), eines NOD-like Rezeptors (NLR), eines RIG I-like (RLR) Rezeptors, eines C-Typ Lectin Rezeptors (CLR), eines cytosolischen dsDNA Sensors (CDSS), eines Scavenger Rezeptors, eines Mannose-binding lectin 2 (MBL-2) Rezeptors, eines Glucan-Rezeptors und Kombinationen davon.

11. Das Testsystem gemäß einem der vorstehenden Ansprüche, wobei die mindestens eine, immobilisierte Pyrogen-Bindedomäne die Pyrogen-Bindedomäne eines TLRs ist.

12. Das Testsystem gemäß einem der vorstehenden Ansprüche, wobei die mindestens eine, immobilisierte Pyrogen-Bindedomäne die Pyrogen-Bindedomäne eines oder mehrerer TLRs ausgewählt aus der Gruppe bestehend aus TLR 1, TLR 2, TLR 3, TLR 4, TLR 5, TLR 6, TLR 7, TLR 8, TLR 9 und Heterodimeren davon ist.

13. Das Testsystem gemäß einem der vorstehenden Ansprüche, wobei das nachzuweisende Pyrogen mindestens eines ausgewählt aus der Gruppe bestehend aus bakteriellen Lipoproteinen, bakteriellen Lipopeptiden, bakteriellen Peptidoglykanen, bakteriellen Lipoteichonsäuren, Zymosan aus Hefen, doppelsträngiger Virus-RNA, bakteriellen Lipopolysacchariden (LPS), bakteriellem Flagellin, nicht methylierter CpG-enthaltender bakterieller oder viraler DNA, einzelsträngiger Virus-RNA und menschlichem Heatshockprotein 60 ist.

14. Das Testsystem gemäß einem der vorstehenden Ansprüche, wobei die immobilisierte Pyrogen-Bindedomäne die Pyrogen-Bindedomäne von TLR4 ist und in der Probe enthaltene Lipopolysaccharide (LPS) nachgewiesen werden.

15. Verfahren zum Nachweis mindestens eines in einer flüssigen Probe enthaltenen Pyrogens umfassend die Verfahrensschritte:
i) Bereitstellen eines Testsystems gemäß einem der Ansprüche 1 bis 14,
ii) Inkontaktbringen einer flüssigen Probe mit dem Assayträger und
iii) Auswerten der Farbreaktion.

16. Verfahren gemäß Anspruch 15, wobei der Assayträger ein kapillarflussfähiges Substrat ist und die flüssige Probe in Schritt ii) auf den Probeaufgabebereich a) aufgebracht wird und Bedingungen bereitgestellt und aufrechterhalten werden, die zu einem Kapillarfluss im Substrat vom Probeaufgabebereich a) bis zum Analysebereich c), bevorzugt bis zum Kontrollbereich d), führen.

17. Verfahren gemäß einem der Ansprüche 15 oder 16, wobei durch das Aufbringen der flüssigen Probe ein in der Probe enthaltenes Pyrogen durch Kapillarfluss in Richtung des Reaktionsbereichs b) transportiert wird, dort an die immobilisierte Pyrogen-Bindedomäne des PRRs bindet und dadurch den markierten Liganden von der immobilisierten Pyrogen-Bindedomäne verdrängt, welcher durch den Kapillarfluss weiter zum Analysebereich c) transportiert, dort durch das immobilisierte Ligandenfängerprotein gebunden und nachgewiesen wird.

18. Verfahren gemäß einem der vorstehenden Ansprüche, wobei eine im Kontrollverbindungsbereich b') adsorbierte Kontrollverbindung durch den Kapillarfluss bis zum Kontrollbereich d) transportiert, dort durch das immobilisierte Kontrollverbindungsfängerprotein gebunden und nachgewiesen wird.

## Claims

1. Competitive immunoassay test system for detecting at least one pyrogen contained in a liquid sample, comprising an assay carrier having at least one immobilized pyrogen binding domain of at least one pattern recognition receptor (PRR) with at least one labeled, displaceable ligand, wherein the displacement of the labeled ligand from the pyrogen binding domain of the PRR is indicated by the pyrogen contained in the sample by a color reaction of the labeling, and wherein the assay carrier has at least one immobilized ligand capture protein for binding the displaced, labeled ligand and wherein the binding of the ligand to the ligand capture protein is indicated by a color reaction of the labeling.

2. The test system according to claim 1, wherein the labeled, displaceable ligand and the at least one immobilized pyrogen binding domain are configured such that the color reaction takes place by displacement of the labeled ligand from the pyrogen binding domain.

3. The test system according to one of claims 1 to 2, wherein the assay carrier is a microtiter plate well, a reagent container, a test tube or a substrate capable of capillary flow.

4. The test system according to claim 3, wherein the substrate that is capable of capillary flow has the following areas in the direction of capillary flow each spaced apart from one another:
a) a sample application area,
b) a reaction area having the at least one immobilized pyrogen binding domain of at least one PRR with the at least one labeled, displaceable ligand and
c) an analysis area having the at least one immobilized ligand capture protein for binding the displaced labeled ligand.

5. The test system according to one of claims 3 or 4, further comprising
b') a control compound area having at least one adsorbed, labeled control compound that does not bind to the pyrogen binding domain of the PRR or the ligand capture protein and
d) a control area having at least one immobilized control compound capture protein for binding the at least one labeled control compound.

6. The test system according to one of claims 3 to 5, wherein the areas a), b), c) and d) are each spaced apart from one another and the areas b) and b') overlap each other or are spaced apart from each other.

7. The test system according to one of the preceding claims, wherein the displaceable ligand and, if present, the control compound are labeled with the same or different color labeling(s) selected from the group consisting of fluorescence dyes, in particular fluorescein isothiocyanate (FITC), fluorescein, rhodamine, ATTO dyes, Texas Red, phycoerythrin derivatives, cyanine fluorescence dyes; colored latex particles, soot particles, magnetic particles, quantum dots, colloidal selenium particles, gold nanoparticles, gold clusters, colloidal gold particles; enzymes, in particular horseradish peroxidase (HRP), alkaline phosphatase (AP), glucose oxidase (GOx), β-D-galactosidase (β-Gal), glucose 6-phosphate dehydrogenase (G6PD); chemiluminescent compounds, in particular acridinium ester, acridinium sulfonamide, isoluminol; streptavidin, avidin and biotin.

8. The test system according to one of the preceding claims, wherein the liquid sample is a clinical sample, a medical-technical sample, a pharmaceutical sample, a food technology sample or an environmental sample.

9. The test system according to one of the preceding claims, wherein the clinical sample is of human or animal origin.

10. The test system according to one of the preceding claims, wherein the at least one immobilized pyrogen binding domain is selected from the group consisting of the pyrogen binding domains of a toll-like receptor (TLR), a NOD-like receptor (NLR), a RIG-I-like receptor (RLR), a C-type lectin receptor (CLR), a cytosolic dsDNA sensor (CDSS), a scavenger receptor, a mannose-binding lectin 2 (MBL-2) receptor, a glucan receptor and combinations thereof.

11. The test system according to one of the preceding claims, wherein the at least one immobilized pyrogen binding domain is the pyrogen binding domain of a TLR.

12. The test system according to one of the preceding claims, wherein the at least one immobilized pyrogen binding domain is the pyrogen binding domain of one or more TLRs selected from the group consisting of TLR 1, TLR 2, TLR 3, TLR 4, TLR 5, TLR 6, TLR 7, TLR 8, TLR 9 and heterodimers thereof.

13. The test system according to one of the preceding claims, wherein the pyrogen to be detected is at least one selected from the group consisting of bacterial lipoproteins, bacterial lipopeptides, bacterial peptidoglycans, bacterial lipoteichoic acids, zymosan of yeast, double-stranded virus RNA, bacterial lipopolysaccharides (LPS), bacterial flagellin, unmethylated bacterial or viral DNA containing CpG, single-strand virus RNA and human heat shock protein 60.

14. The test system according to one of the preceding claims, wherein the immobilized pyrogen binding domain is the pyrogen binding domain of TLR4 and lipopolysaccharides (LPS) contained in the sample are detected.

15. Method for detecting at least one pyrogen contained in a liquid sample comprising the following process steps:
i) providing a test system according to one of claims 1 to 14,
ii) bringing a liquid sample into contact with the assay carrier and
iii) analyzing the color reaction.

16. Method according to claim 15, wherein the assay carrier is a substrate capable of capillary flow and the liquid sample is applied to the sample application area a) in step ii) and conditions are provided and maintained, which produce a capillary flow in the substrate from the sample application area a) to the analysis area c), preferably to the control area d).

17. Method according to one of claims 15 or 16, wherein, by applying the liquid sample, a pyrogen contained in the sample is transported by capillary flow in the direction of the reaction area b), binds there to the immobilized pyrogen binding domain of the PRR and thereby displaces the labeled ligand from the immobilized pyrogen binding domain, which is transported further to the analysis area c), is bound there by the immobilized ligand capture protein and detected.

18. Method according to one of the preceding claims, wherein a control compound adsorbed in the control compound area b') is transported by the capillary flow to the control area d), bound there by the immobilized control compound capture protein and detected.

## Revendications

1. Système d'essai de dosage immunologique compétitif pour la détection d'au moins un pyrogène contenu dans un échantillon liquide, comprenant un support de dosage, présentant au moins un domaine de liaison de pyrogène immobilisé d'au moins un récepteur de reconnaissance de motifs (PRR, pattern recognition receptor) avec au moins un ligand marqué, pouvant être déplacé, dans lequel le déplacement du ligand marqué à partir du domaine de liaison de pyrogène du PRR est indiqué par le pyrogène contenu dans l'échantillon par une réaction colorée du marqueur et dans lequel le support de dosage présente au moins une protéine réceptrice de ligand immobilisée pour la liaison du ligand déplacé marqué et la liaison du ligand à la protéine réceptrice de ligand est indiquée par une réaction colorée du marqueur.

2. Système d'essai selon la revendication 1, dans lequel le ligand marqué, pouvant être déplacé, et l'au moins un domaine de liaison de pyrogène immobilisé sont conçus de telle manière que la réaction colorée a lieu lors du déplacement du ligand marqué à partir du domaine de liaison de pyrogène.

3. Système d'essai selon l'une des revendications 1 à 2, dans lequel le support de dosage est une cuvette d'une plaque de microtitrage, un flacon de réaction, un tube à essai ou un substrat apte à un flux capillaire.

4. Système d'essai selon la revendication 3, dans lequel le substrat apte à un flux capillaire présente, dans le sens du flux capillaire, les régions suivants, séparées respectivement les uns des autres :
a) une région d'application d'échantillon,
b) une région de réaction, présentant l'au moins un domaine de liaison de pyrogène immobilisé d'au moins un PRR avec l'au moins un ligand marqué, pouvant être déplacé, et
c) une région d'analyse, présentant l'au moins une protéine réceptrice de ligand immobilisée pour la liaison du ligand déplacé, marqué.

5. Système d'essai selon l'une des revendications 3 ou 4, comprenant en outre
b') une région de composé de contrôle présentant au moins un composé de contrôle adsorbé, marqué, qui ne se lie pas au domaine de liaison de pyrogène du PRR ou à la protéine réceptrice de ligand, et
d) une région de contrôle, présentant au moins une protéine réceptrice de composé de contrôle immobilisée pour la liaison de l'au moins un composé de contrôle marqué.

6. Système d'essai selon l'une des revendications 3 à 5, dans lequel les régions a), b), c) et d) sont respectivement séparés les uns des autres et les régions b) et b') se superposent ou sont espacés l'un de l'autre.

7. Système d'essai selon l'une des revendications précédentes, dans lequel le ligand, pouvant être déplacé, et le composé de contrôle, s'il est présent, sont marqués avec des marqueurs de couleur identiques ou différents choisis dans le groupe constitué des colorants de fluorescence, notamment l'isothiocyanate de fluorescéine (FITC), la fluorescéine, la rhodamine, des colorants ATTO, le rouge Texas, des dérivés de phycoérythrine, les colorants de fluorescence de cyanine ; de particules de latex colorées, de particules de suie, de particules magnétiques, de points quantiques, de particules de sélénium colloïdales, de nanoparticules d'or, d'agglomérats d'or, de particules d'or colloïdales ; d'enzymes, notamment de peroxydase de raifort (HRP), de phosphatase alcaline (AP), de glucoseoxidase (GOx), de β-D-galactosidase (β-Gal), de glucose 6-phosphate déshydrogénase (G6PD) ; de composés chimiluminescents, notamment d'ester d'acridinium, de sulfonamide d'acridinium, d'isoluminol ; de streptavidine, d'avidine et de biotine.

8. Système d'essai selon l'une des revendications précédentes, dans lequel l'échantillon liquide est un échantillon clinique, un échantillon médico-technique, un échantillon pharmaceutique, un échantillon de la technologie des produits alimentaires ou un échantillon environnemental.

9. Système d'essai selon l'une des revendications précédentes, dans lequel l'échantillon clinique est d'origine humaine ou animale.

10. Système d'essai selon l'une des revendications précédentes, dans lequel l'au moins un domaine de liaison de pyrogène immobilisé est choisi dans le groupe constitué des domaines de liaison de pyrogènes d'un récepteur Toll-like (TLR), d'un récepteur NOD-like (NLR), d'un récepteur RIG I-like (RLR), d'un récepteur de lectine de type C (CLR), d'un capteur de dsADN cytosolisique (CDSS), d'un récepteur scavenger, d'un récepteur de lectine 2 liant le mannose (MBL-2), d'un récepteur de glucane et de combinaisons de ceux-ci.

11. Système d'essai selon l'une des revendications précédentes, dans lequel l'au moins un domaine de liaison de pyrogène immobilisé est le domaine de liaison de pyrogène d'un TLR.

12. Système d'essai selon l'une des revendications précédentes, dans lequel l'au moins un domaine de liaison de pyrogène immobilisé est le domaine de liaison de pyrogène d'un ou de plusieurs TLR choisis dans le groupe constitué des TLR 1, TLR 2, TLR 3, TLR 4, TLR 5, TLR 6, TLR 7, TLR 8, TLR 9 et d'hétéro dimères de ceux-ci.

13. Système d'essai selon l'une des revendications précédentes, le pyrogène à détecter est au moins l'un choisi dans le groupe constitué de lipoprotéines bactériennes, de lipopeptides bactériens, de peptidoglycanes bactériens, d'acides lipotéichoïques bactériens, de zymosane provenant de levures, d'ARN de virus à double brin, de lipopolysaccharides bactériens (LPS), d'un flagelle bactérien, d'ADN bactérien ou viral non méthylé contenant de CPG, d'ARN de virus à brin unique et de la protéine humaine de choc thermique 60.

14. Système d'essai selon l'une des revendications précédentes, dans lequel le domaine de liaison de pyrogène immobilisé est le domaine de liaison de pyrogène de TLR4 et des lipopolysaccharides (LPS) contenus dans l'échantillon sont détectés.

15. Procédé de détection d'au moins un pyrogène contenu dans un échantillon liquide comprenant les étapes de procédé :
i) fourniture d'un système d'essai selon l'une des revendications 1 à 14,
ii) mise en contact d'un échantillon liquide avec le support de dosage, et
iii) exploitation de la réaction colorée.

16. Procédé selon la revendication 15, dans lequel le support de dosage est un substrat apte au flux capillaire et l'échantillon liquide est appliqué sur la région d'application d'échantillon a) dans l'étape ii) et des conditions sont mises en place et maintenues qui mènent à un flux capillaire dans le substrat à partir de la région d'application de l'échantillon a) jusqu'à la région d'analyse c), de préférence, jusqu'à la région de contrôle d).

17. Procédé selon l'une des revendications 15 ou 16, dans lequel, par l'application de l'échantillon liquide, un pyrogène contenu dans l'échantillon est transporté par un flux capillaire en direction de la région de réaction b), se lie au domaine de liaison de pyrogène du PRR immobilisé à cet endroit et déplace ainsi le ligand marqué du domaine de liaison de pyrogène immobilisé, lequel est transporté par le flux capillaire jusqu'à la région d'analyse c), est lié à cet endroit par la protéine réceptrice de ligand immobilisée et est détecté.

18. Procédé selon l'une des revendications précédentes, dans lequel, un composé de contrôle adsorbé dans la région de composé de contrôle b') est transporté par le flux capillaire jusqu'à la région de contrôle d), est lié à cet endroit par la protéine réceptrice de composé de contrôle immobilisé et est détecté.


### SEQUENCE LISTING

<110> Fraunhofer-Gesellschaft zur Förderung der angewandten Forschung e.V.
<120> Kompetitives Immunassay-Testsystem zum Nachweis eines Pyrogens
<130> 205788
<160> 7
<170> PatentIn version 3.5
<210> 1
   <211> 20
   <212> PRT
   <213> Artificial Sequence
<220>
   <223> Peptide
<400> 1
   Lys Gly Glu Thr Val Asn Thr Thr Ile Ser Phe Ser Phe Lys Gly Ile
   1               5                   10                  15
   Lys Phe Ser Lys
               20
<210> 2
   <211> 20
   <212> PRT
   <213> Artificial Sequence
<220>
   <223> Peptide
<400> 2
   Lys Met Gln Tyr Pro Ile Ser Ile Asn Val Asn Pro Cys Ile Glu Leu
   1               5                   10                  15
   Lys Gly Ser Lys
               20
<210> 3
   <211> 20
   <212> PRT
   <213> Artificial sequence
<220>
   <223> Peptide
<400> 3
   Gly Leu Leu His Ile Phe Tyr Ile Pro Arg Arg Asp Leu Lys Gln Leu
   1               5                   10                  15
   Tyr Phe Asn Leu
               20
<210> 4
   <211> 20
   <212> PRT
   <213> Artificial sequence
<220>
   <223> Peptide
<400> 4
   Lys Arg Lys Glu Val Ile Cys Arg Gly Ser Asp Asp Asp Tyr Ser Phe
   1               5                   10                  15
   Cys Arg Ala Leu
               20
<210> 5
   <211> 10
   <212> PRT
   <213> Artificial sequence
<220>
   <223> Peptide
<400> 5
   Phe Met Met Leu Gly Gly Leu Val Arg Ile
   1               5                   10
<210> 6
   <211> 9
   <212> PRT
   <213> Artificial sequence
<220>
   <223> Peptide
<400> 6
   Arg Met Met Trp Phe Gly Ile Met Val
   1               5
<210> 7
   <211> 22
   <212> PRT
   <213> Artificial sequence
<220>
   <223> Peptide
<400> 7
   Ser Gly Ser Pro Glu Glu Met Leu Phe Cys Leu Glu Phe Val Ile Leu
   1               5                   10                  15
   His Gln Pro Asn Ser Asn
               20